(19) Europäisches Patentamt
European Patent Office
Office européen des brevets

(11) Publication number: 0 516 095 A2

# EUROPEAN PATENT APPLICATION

(21) Application number: 92108971.0

(22) Date of filing: 27.05.92

(51) Int. Cl.5: G01N 33/53, G01N 33/543, G01N 33/52, G01N 33/58

(30) Priority: 29.05.91 JP 126189/91

(43) Date of publication of application:
02.12.92 Bulletin 92/49

(84) Designated Contracting States:
DE FR GB NL

(71) Applicant: MOCHIDA PHARMACEUTICAL CO., LTD.
7, Yotsuya 1-chome
Shinjuku-ku Tokyo 160(JP)

(72) Inventor: Yamauchi, Tadakazu, c/o MOCHIDA PHARMACEUT.CO.,LTD
7, Yotsuya 1-chome, Shinjuku-ku
Tokyo(JP)
Inventor: Sugihara, Keisuke, c/o MOCHIDA PHARMACEUT.CO.,LTD
7, Yotsuya 1-chome, Shinjuku-ku
Tokyo(JP)
Inventor: Sato, Hiroshi, c/o MOCHIDA PHARMACEUT.CO.,LTD
7, Yotsuya 1-chome, Shinjuku-ku
Tokyo(JP)
Inventor: Kanamori, Toshinori, c/o MOCHIDA PHARMACEUT.CO.LTD
7, Yotsuya 1-chome, Shinjuku-ku
Tokyo(JP)

(74) Representative: Patentanwälte Grünecker, Kinkeldey, Stockmair & Partner
Maximilianstrasse 58
W-8000 München 22(DE)

(54) Process and device for specific binding assay.

(57) An assay process and a device useful for the practice of the assay process, effective for high sensitivity semi-quantitative assay, in which assay results can be obtained by simply loading a sample as it is and any desired detection sensitivity can be selected at will without causing decrease (or increase) in the maximum intensity of signals.

Particularly, this invention provides a process for specific binding assay in which a substance in a test sample to be assayed is detected qualitatively or quantitatively making use of specific binding reaction, which comprises including a specified substance in an assay system and, by the effect of the presence of the specified substance, decreasing (or increasing) amounts of a labeled material to be measured as an index of the amount of the substance to be assayed. It also provides a chromatography type specific binding assay device which comprises a means for loading a test sample, a means for locating specific binding substances, a means for locating a detecting element and a means for absorbing liquid materials in that order, wherein a specific binding substance and a labeled specific binding substance are included in the means for locating specific binding substances and a detecting element on which a specific binding substance is immobilized is included in the means for locating a detecting element.

## Fig. 1

EP 0 516 095 A2

FIELD OF THE INVENTION

This invention relates to an assay process by which a substance in a test sample can be measured with any desired detection sensitivity selected at will without requiring dilution of the sample. It also relates to an assay device which is useful for the practice of the assay process.

BACKGROUND OF THE INVENTION

Immune reaction of antigens with antibodies specific to the antigens can be regarded as one of specific binding reactions. The immune reaction has been applied broadly to the measurement of trace substances in the living body, with illustrative examples of such applications including quantitative detection methods such as radioimmunoassay, fluoroimmunoassay, chemiluminescence immunoassay, enzyme immunoassay and the like and qualitative detection methods such as hemagglutination test, latex agglutination test and the like. These methods have been put into practical use in the clinical field for the diagnosis of various diseases and the monitoring of therapeutic effects.

The development of such immunoassays has been highly advanced, with recently developed examples including various simple assay processes and kits for use therein disclosed for instance in JP-A 01-299464 (the term "JP-A" as used herein means an "unexamined published Japanese patent application"), JP-W 61-502214 (the term "JP-W" as used herein means an "unexamined laid-open PCT application in Japanese national phase") and the like. When practical application of an immunoassay to the clinical field is taken into consideration, it is desirable to use a process or a kit by which substances in test samples can be measured in quantitative or semi-quantitative way through simple observation by the naked eye without using measuring instruments.

In the case of the conventional immunological methods for the detection of trace substances in the living body, including the aforementioned simple assay methods, attention has been directed solely to the improvement of detection sensitivities because of the extremely small quantities of the substances to be assayed. Because of this, test samples containing substances to be assayed are used as they are or after their concentration, except for an especially necessary case. In this instance, the term "an especially necessary case" means a case in which the amount of a test sample is so small that it is necessary to dilute the test sample.

However, it is desirable to avoid such concentration or dilution steps during clinical inspection, because these steps have a possibility of infection to the persons who engage in the inspection especially when a sample to be tested contains HB virus or the like.

In order to control detection sensitivity without employing concentration or dilution step of samples, various techniques have been proposed in which the quantity (concentration) of immobilized substances or labeled substances that constitute an immunoassay kit is changed or their binding affinities for substances to be assayed are changed. Illustrative examples of such techniques are disclosed for instance in JP-A 60-192261 (semi-quantitative), JP-A 01-245157 (semi-quantitative), JP-W 01-503174 (qualitative), JP-A 01-244370 (qualitative and quantitative) and the like.

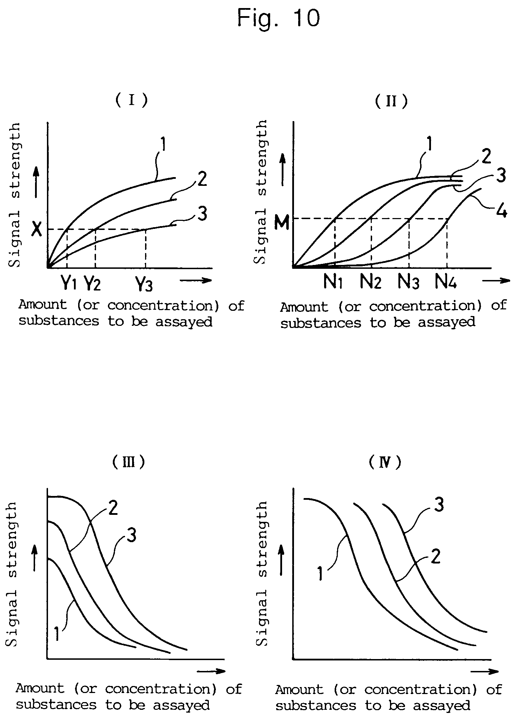

However, control of the detection sensitivity by such prior art techniques entails problems which will be described in the following using graphs shown in Fig. 10.

Fig. 10 (I) is a graph schematically showing a relationship between the amount (or concentration) of a substance to be assayed and the signal intensity in a sandwich type immunoassay. In this figure, curves 1 to 3 indicate the use of different amounts of a labeled material in the assay system, and the detection sensitivity is highest in curve 1, followed by curve 2 and curve 3 in that order.

Provided that qualitative judgement is made in this assay system using a signal intensity X as a boundary on the presence (signal intensity $\geq$ X) or absence (signal intensity < X) of a substance to be assayed, presence of the substance to be assayed is judged positive when the amount of the substance is Y1 or more or judged negative when the amount of the substance is less than Y1 in the case of the use of a kit in which the detection sensitivity is controlled to obtain the curve 1. In the case of a kit in which the detection sensitivity is controlled to obtain the curve 2 or curve 3, presence or absence of a substance to be assayed is judged based on the amount of the substance Y2 or Y3 as a criterional boundary.

In this instance, reduction of the detection sensitivity (increment of the amount of a substance to be assayed as a criterional boundary) means control of the detection sensitivity to obtain the curve 2 or 3. In that case, as is evident from the figure, decrease in the maximum intensity of signals which reflect the amount of a substance to be assayed in a test sample is unavoidable, thus entailing inaccuracy of the judgement of the results. Another disadvantage of this case is that the area of a zone phenomenon shifts to low concentration side of a substance to be assayed.

3

Contrary to the case of the sandwich type immunoassay, signal intensity in a competition type immunoassay decreases as the amount (or concentration) of a substance to be assayed increases. In the competition type immunoassay, the amount of a labeled material which competes with the substance to be assayed is increased as a means to reduce the detection sensitivity. Such a means is shown schematically in Fig. 10 (III) in which the detection sensitivity is most high in curve 1, followed by curve 2 and curve 3 in that order. As is evident from the figure, increase in the maximum intensity of signals is unavoidable when the detection sensitivity is reduced, thus entailing inaccuracy of the judgement of the results.

As has been described in the foregoing, though various simple assay methods and kits have been developed in the field of immunoassays, virtually nothing is known about a simple assay method in which the maximum intensity of signals to be measured does not decrease and the area of a zone phenomenon does not change when detection sensitivity is controlled corresponding to the amount of a substance to be assayed in a test sample and which, without employing a step for the dilution of the sample prior to the commencement of the assay process or prior to the application of the sample to an assay kit, can show detection results similar to the case of the use of a diluted test sample, as well as about a kit which is useful for the practice of such a simple assay method and by which a substance to be assayed in a test sample can be measured qualitatively or quantitatively by simply loading the sample.

SUMMARY OF THE INVENTION

In view of the above, it therefore becomes an object of the present invention to provide an assay process which is especially effective for semi-quantitative assay at a high sensitivity, by which assay results similar to the case of the use of a diluted test sample can be obtained even when the sample is used as it is, and in which any desired detection sensitivity can be selected at will without causing changes in the maximum intensity of signals. Another object of the present invention is to provide an assay device useful for the practice of the assay process.

Particularly, in accordance with a first aspect of the present invention, there is provided a process for specific binding assay in which a substance in a test sample to be assayed is detected qualitatively or quantitatively making use of specific binding reaction, which comprises including a specified substance in an assay system and, by the effect of the presence of the specified substance, decreasing amounts of a labeled material to be measured as an index of the amount of the substance to be assayed.

According to a second aspect of the present invention, there is provided a process for specific binding assay in which a substance in a test sample to be assayed is detected qualitatively or quantitatively making use of specific binding reaction, which comprises including a specified substance in an assay system and, by the effect of the presence of the specified substance, increasing amounts of a labeled material to be measured as an index of the amount of the substance to be assayed.

According to a third aspect of the present invention, there is provided a chromatographic specific binding assay device which is useful for the practice of the specific binding assay process of the first and second aspects of the present invention, that comprises a means (A) for loading a test sample, a means (B) for locating specific binding substances, a means (C) for locating a detecting element and a means (D) for absorbing liquid materials in that order.

Other objects and advantages of the present invention will be made apparent as the description progresses.

BRIEF DESCRIPTION OF THE DRAWINGS

Figs. 1 to 9 are schematic representations for use in the description of the specific binding assay process of the present invention.

Fig. 10 is a graph showing relationships between the amount of a substance to be assayed and the signal intensity in the inventive and current specific binding assays.

Figs. 11 to 14 are graphs showing preferred examples of the specific binding assay device of the present invention.

Figs. 15 and 16 are schematic representations showing results of the specific binding assay of the present invention.

Fig. 17 is a graph showing a preferred example of the specific binding assay device of the present invention.

Figs. 18 and 19 are graphs showing modified examples of the specific binding assay device of the present invention.

Fig. 20 is a graph showing another example of the specific binding assay device for use in the practice of the specific binding assay process of the present invention.

Fig. 21 is a schematic representation showing the results of Example 7.

Fig. 22 is a schematic representation showing the results of Example 8.

Fig. 23 is a schematic representation showing the results of Example 9.

In these figures, A indicates a means for loading a test sample, B indicates a means for locating specific binding substances, C indicates a means for locating a detecting element, D indicates a means for absorbing liquid materials, E indicates a detecting element and F indicates a support.

## DETAILED DESCRIPTION OF THE INVENTION

Firstly, technical terms used in the specification of this invention are explained.

The term "substance to be assayed" as used herein means a substance which is detected qualitatively or quantitatively by the process or the device of the present invention. Examples of such substances include various proteins and peptides which are functionally capable of acting as antigens or antibodies, nucleic acids, effector molecules, receptor molecules, enzymes, inhibitors, avidin, biotin, sugar chain-containing compounds, lectins and the like. More illustratively, such substances include HBs antigen, anti-HBs antibody, estriol ($E_3$), $\alpha$-fetoprotein (AFP), human chorionic gonadotropin (hCG), luteinizing hormone (LH), carcinoembryonic antigen (CEA), $\beta_2$ microglobulin ($\beta_2$ m), ferritin, human placental lactogen (hPL) and the like.

The term "analogue of a substance to be assayed" as used herein means a substance which shows similar behavior to the substance to be assayed in a binding reaction with a certain substance. Illustrative examples include various substances which are analogous to the substances to be assayed.

The term "test sample" as used herein means a liquid material which contains or probably contains a substance to be assayed, with illustrative examples including urine, blood plasma, sera, whole blood, saliva, tear fluid, cerebrospinal fluid and the like. Also included are solid or gel substances dissolved in liquid materials such as buffers and the like.

The term "specific binding substance" as used herein means a substance which has a specific affinity for a certain substance that has a specified structure. In this specification, a specific binding substance I which has a specific affinity for the substance to be assayed or an analogue thereof and another specific binding substance II that has a specific affinity for the specific binding substance I are described. Examples of combinations of the substance having a specified structure with the specific binding substance I include antigens with corresponding antibodies, a nucleic acid sequence with its complementary sequence, effector molecules with receptor molecules, enzymes with inhibitors, avidin with biotin, sugar chain-containing compounds with lectins and the like combinations. Examples of combinations of the specific binding substance I with the specific binding substance II include the anti-antibody with antibody which is specific for an antigen.

The term "to interfere" as used herein means that a substance interferes with a set of binding reaction, for example a reaction of a substance to be assayed with a specific binding substance I, through a certain means such as steric hindrance, competitive binding or the like.

The term "labeling substance" as used herein means a substance which can show the presence or the concentration of a substance to be assayed in a test sample directly or indirectly. Illustrative examples of the direct labeling substance include: various types of dyes and pigments; radioactive materials such as $^{125}I$, $^{32}P$, $^{3}H$, $^{14}C$ and the like; chemiluminescent materials such as luminol derivatives, acridinium esters and the like; biological luminescent materials such as firefly luciferin, *Cypridina* luciferin and the like; fluorescent materials such as fluorescein, rhodamine and the like; and colored latex, gold colloid and the like. Illustrative examples of the indirect labeling substance include various types of enzymes.

When an enzyme is used as the labeling substance, a substrate or a chromogen is treated with the enzyme, and resulting changes in the properties of the substrate or chromogen, such as color density, luminescence, fluorescence and the like, are measured. Illustrative examples of the combination of enzymes with substrates (and chromogens) include: combination of enzymes with coloring substrates such as horseradish peroxidase with $H_2O_2$ (and 3,3',5,5'-tetramethylbenzidine), alkaline phosphatase with 5-bromo-4-chloro-3-indolyl phosphate, galactosidase with 2-nitrophenyl-$\beta$-D-galactoside and the like; combination of enzymes with luminescent substrates such as alkaline phosphatase with 3-(2'-spiroadamantene)-4-methoxy-4-(3''-phosphoryloxyphenyl-1,2-dioxetane (AMPPD), horseradish peroxidase with luminol, luciferase with luciferin and the like; and combination of enzymes with fluorescent substrates such as alkaline phosphatase with umbelliferyl phosphate, horseradish peroxidase with p-hydroxyphenyl propionate and the like.

The term "signal intensity" as used herein means color density, radioactivity, luminescence, fluorescence or the like directly or indirectly shown by the labeling substance.

5

The term "labeled material" as used herein means a material which contains the labeling substance.

The term "means for loading test sample" as used herein means a part where a test sample to be assayed is loaded, and the term "means for locating specific binding substances" means a part where the aforementioned specific binding substance and the like are located.

The term "detecting element" as used herein means a part where a substance to be assayed, an analogue of the substance to be assayed, a specific binding substance or the like is immobilized and signals originated from the labeling substance are detected, and the term "means for locating a detecting element" means a part which includes the detecting element and its peripheral area. In this instance, the means for locating a detecting element may be the same as or different from the detecting element.

The term "means for absorbing liquid materials" as used herein means a part where liquid materials such as a loaded test sample and the like are absorbed and maintained.

The following describes a first, a second and a third aspects of the present invention making use of the thus defined terms.

According to the first aspect of the present invention, there is provided a process for specific binding assay in which a substance in a test sample to be assayed is detected qualitatively or quantitatively making use of specific binding reaction, which comprises including a specified substance in an assay system and, by the effect of the presence of said specified substance, decreasing amounts of a labeled material to be measured as an index of the amount of the substance to be assayed.

In this instance, the term "specified substance" means a substance which corresponds to the amount or the concentration of a substance to be assayed and contributes to the regulation of the amount of a labeled material to be measured. A suitable specified substance may be selected depending on each assay system from, for example, specific binding substances having specific affinities for substances to be assayed, analogues of substances to be assayed and the like.

Also, a suitable labeled material may be selected depending on each assay system. Illustrative examples of the labeled material include a material formed through labeling of a specific binding substance which has a specific affinity for a substance to be assayed, a material formed through labeling of a substance which binds to a specific binding substance having a specific affinity for a substance to be assayed, a material formed through labeling of a substance to be assayed, a material formed through labeling of an analogue of a substance to be assayed and the like. In this instance, the term "formed through labeling" means a state in which a labeling substance is bound to a certain substance directly or indirectly.

The following describes illustrative examples of the first aspect of the present invention on the basis of the figures attached hereto.

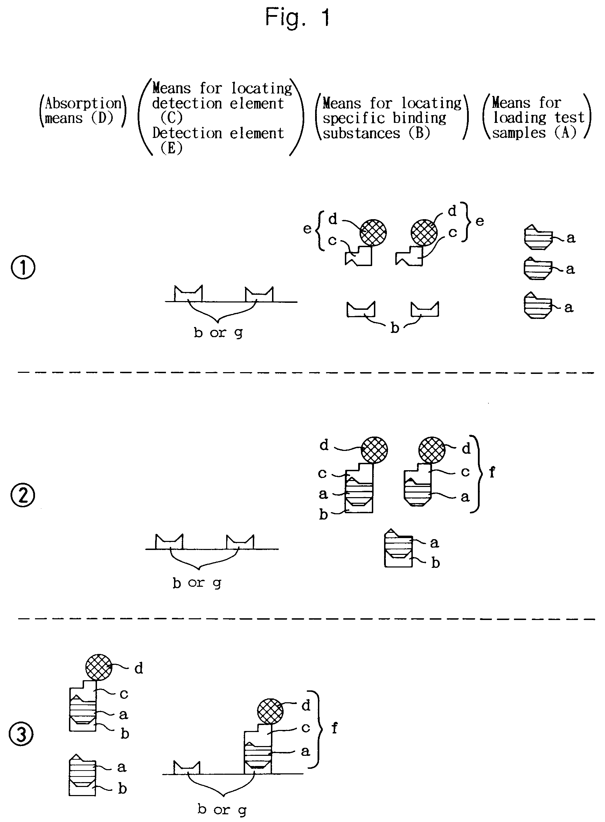

Firstly, an example of the first aspect of the present invention is explained using Fig. 1.

The example shown in Fig. 1 is an assay system in which the specified substance is a specific binding substance (b) having a specific affinity for a substance (a) to be assayed, and the labeled material is a labeled specific binding substance (e) which is a compound resulting from binding of a labeling substance (d) to a specific binding substance (c) that has a specific affinity for the substance (a) to be assayed but does not interfere with the binding reaction of the substance (a) to be assayed with the specific binding substance (b).

In Fig. 1, the assay system is effected by a process which comprises the steps of:

allowing a substance (a) to be assayed to undergo binding reaction simultaneously or successively with a specific binding substance (b) and/or with a labeled specific binding substance (e);

allowing a binding substance (f), which is a compound resulting from the above binding reaction of said substance (a) to be assayed with said labeled specific binding substance (e), to undergo binding reaction with said specific binding substance (b) in an immobilized form or with an immobilized specific binding substance (g) that does not interfere with said labeled specific binding substance (e) but interferes with said specific binding substance (b) at the time of binding reaction with said substance (a) to be assayed, thereby immobilizing said binding substance (f); and reading off signals originated from the labeling substance (d) in the immobilized binding substance (f). This assay process will be called "process 1" hereinafter.

In this instance, the specific binding substance (b) and the specific binding substance (g) interfere with each other in their binding to the substance (a) to be assayed. For example, when the substance (a) to be assayed is a substance which functions as an antigen, the specific binding substances (b) and (g) may be two different types of antibodies but having a specific affinity for the same antigenic determinant, or they may be two different types having specific affinities for different antigenic determinants but having such a relationship that the specific binding substance (g) cannot bind to the substance (a) to be assayed when the specific binding substance (b) binds to the substance (a) in advance.

Typical examples of the binding conditions of each substance related to the assay system of the process 1 are shown in Fig. 1.

In the process 1, when the substance (a) to be assayed is bound to the soluble form of the specific binding substance (b), the resulting complex cannot bind to the immobilized specific binding substance (b) or (g). Because of this, the labeled specific binding substance (e) is excluded from the assay system when it binds to the substance (a) to be assayed to which the soluble specific binding substance (b) is bound. Degree of such an exclusion (degree of dilution) is controlled mainly by the quantitative ratio of the soluble specific binding substance (b) to the immobilized specific binding substance (b) or (g) and/or by the binding affinity of these substances for the substance (a) to be assayed.

In the process 1, when amounts of the labeled specific binding substance (e) and the immobilized specific binding substance (b) or (g) are fixed to constant levels, the amount of the labeled specific binding substance (e) excluded from the assay system increases as the amount of the soluble specific binding substance (b) is increased, thus resulting in the reduction of the intensity of signals originated from the labeling substance (d) read off after completion of the reaction. In this way, the signal intensity can be controlled to the same level as a case in which the amount of the substance (a) to be assayed is reduced by diluting a test sample.

In consequence, in spite of the use of a test sample as it is, similar effect to the case of the dilution of a test sample can be obtained (to be referred to as "dilution effect" hereinafter), and measurement at a proper signal intensity can be attained at a desired detection sensitivity.

When the specific binding substance (g) is used as the immobilized substance, a proper dilution effect can be obtained by controlling binding affinities of the soluble specific binding substance (b) and the immobilized specific binding substance (g) for the substance (a) to be assayed.

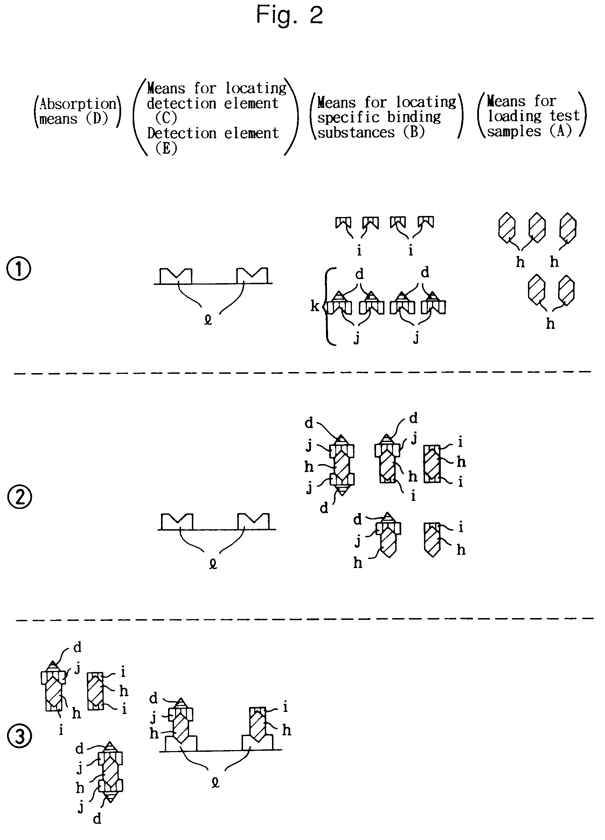

Next, another example of the first aspect of the present invention is explained using Fig. 2.

The example shown in Fig. 2 is an assay system which is useful when a substance (h) to be assayed obviously has a plurality of sites related to one specific binding reaction, in which the specified substance is a specific binding substance (i) having a specific affinity for the substance (h) to be assayed, and the labeled material is a labeled specific binding substance (k) which is a compound resulting from binding of a labeling substance (d) to a specific binding substance (j) that has a specific affinity for the substance (h) to be assayed and interferes with the binding reaction of the substance (h) to be assayed with the specific binding substance (i).

In Fig. 2, the assay system is effected by a process which comprises the steps of:

allowing the substance (h) to be assayed to undergo binding reaction simultaneously or successively with the specific binding substance (i) having a specific affinity for the substance (h) to be assayed, with a labeled specific binding substance (k) which is a compound resulting from binding of the labeling substance (d) to the specific binding substance (j) having a specific affinity for the substance (h) to be assayed and with an immobilized specific binding substance (l) having a specific affinity for the substance (h) to be assayed, thereby immobilizing at least a portion of the substance (h) to be assayed; and

reading off signals originated from the labeling substance (d) in the labeled specific binding substance (k) bound to the immobilized substance (h) to be assayed.

This assay process will be called "process 2" hereinafter.

In this instance, the specific binding substance (i), the specific binding substance (j) which constitutes the labeled specific binding substance (k) and the immobilized specific binding substance (l) may be the same or different substances, provided that they interfere with one another in their binding to the sites related to one specific binding reaction in the substance (h) to be assayed.

Typical examples of the binding conditions of each substance related to the assay system of the process 2 are as shown in Fig. 2.

In the process 2, when the specific binding substance (i) and/or the labeled specific binding substance (k) bind to all of the sites related to one specific binding reaction in the substance (h) to be assayed, the resulting complex cannot bind to the immobilized specific binding substance (l). Because of this, the labeled specific binding substance (k) which binds to the substance (h) to be assayed under such a condition is excluded from the assay system. Degree of such an exclusion is controlled by the quantitative ratio among the specific binding substance (i), the labeled specific binding substance (k) and the immobilized specific binding substance (l) and/or by the binding affinity of these substances for the substance (h) to be assayed. As the results, a dilution effect is obtained similar to the case of the process 1, and measurement at a proper signal intensity can be attained at a desired detection sensitivity.

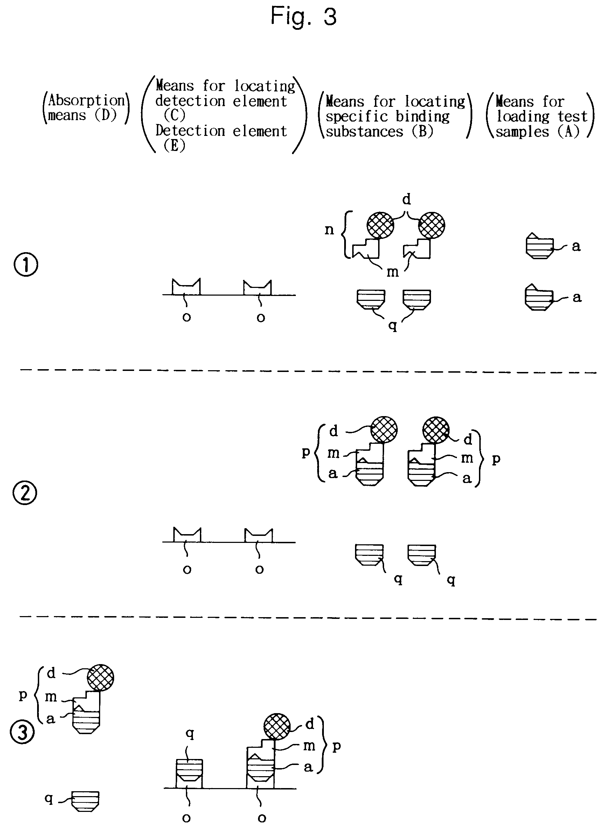

Next, an example of the first aspect of the present invention is explained using Fig. 3.

The example shown in Fig. 3 is an assay system in which the specified substance is an analogue (q) of the substance (a) to be assayed, which interferes with the substance (a) to be assayed at the time of the binding reaction with an immobilized specific binding substance (o), and the labeled material is a labeled specific binding substance (n) which is a compound resulting from binding of a labeling substance (d) to a specific binding substance (m) that has a specific affinity for the substance (a) to be assayed and does not interfere with the binding reaction of the substance (a) to be assayed with the immobilized specific binding substance (o).

In this case, the assay system is effected by a process which comprises the steps of:

allowing the substance (a) to be assayed to undergo binding reaction with the labeled specific binding substance (n); allowing the immobilized specific binding substance (o), which has a specific affinity for the substance (a) to be assayed and is not interfered with the binding reaction of the substance (a) to be assayed with the labeled specific binding substance (n), to undergo binding reaction with the binding substance (p) which is a compound resulting from the binding of the substance (a) to be assayed to the labeled specific binding substance (n) and with an analogue (q) of the substance to be assayed that does not bind to the labeled specific binding substance (n) but binds to the specific binding substance (o), thereby immobilizing said binding substance (p) and said analogue (q); and

reading off signals originated from the labeling substance (d) in the immobilized binding substance (p).

This assay process will be called "process 3" hereinafter.

Typical examples of the binding conditions of each substance related to the assay system of the process 3 are as shown in Fig. 3.

In the process 3, the binding substance (p) and the analogue (q) of the substance to be assayed bind to the immobilized specific binding substance (o) in competition. Because of this, a portion of the labeled specific binding substance (n) is excluded from the assay system as the binding substance (p). Degree of such an exclusion is controlled by the quantitative ratio among the labeled specific binding substance (n), the analogue (q) of the substance to be assayed and the immobilized specific binding substance (o) and/or by the binding affinity of the immobilized specific binding substance (o) for the substance (a) to be assayed and the analogue (q). As the results, a dilution effect is obtained similar to the case of the process 1, and measurement at a proper signal intensity can be attained at a desired detection sensitivity.

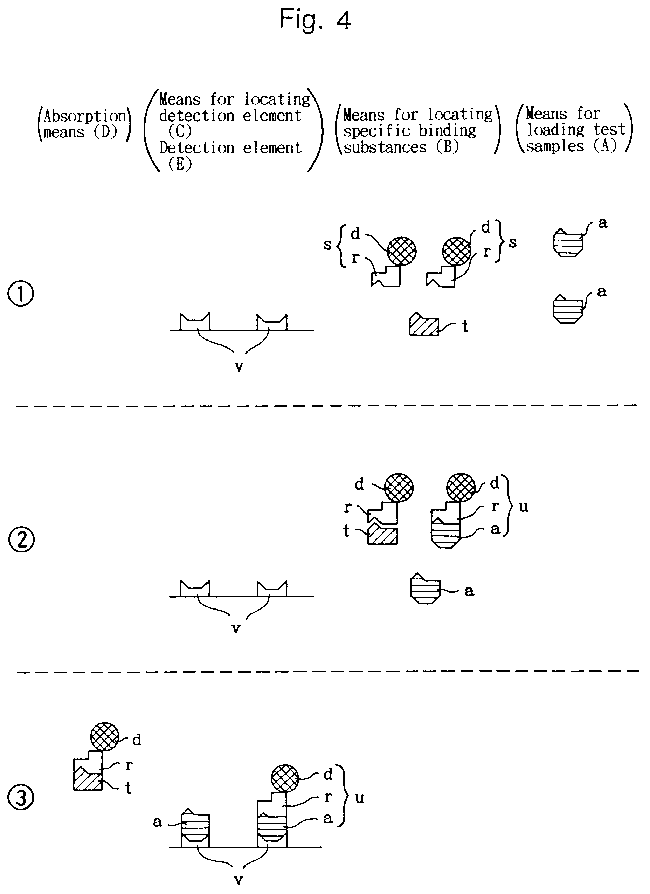

The example shown in Fig. 4 is an assay system in which the specified substance is an analogue (t) of the substance (a) to be assayed, which does not bind to an immobilized specific binding substance (v), and the labeled material is a labeled specific binding substance (s) which is a compound resulting from binding of a labeling substance (d) to a specific binding substance (r) that has specific affinities for the substance (a) to be assayed and the analogue (t).

In this case, the assay system is effected by a process which comprises the steps of:

allowing the labeled specific binding substance (s) to undergo binding reaction simultaneously or successively with the substance (a) in a test sample to be assayed and with the analogue (t) that interferes binding reaction of the labeled specific binding substance (s) with the substance (a) to be assayed;

allowing a binding substance (u) which is a compound resulting from the above binding reaction of the substance (a) to be assayed with the labeled specific binding substance (s) and, if exist, unbound substance (a) to undergo binding reaction with an immobilized specific binding substance (v) that does not interfere with the labeled specific binding substance (s) and does not bind to the analogue (t) of the substance to be assayed at the time of binding reaction with the substance (a) to be assayed, thereby immobilizing the binding substance (u); and

reading off signals originated from the labeling substance (d) in the immobilized binding substance (u).

This assay process will be called "process 4" hereinafter.

Typical examples of the binding conditions of each substance related to the assay system of the process 4 are as shown in Fig. 4.

In the process 4, the substance (a) to be assayed and the analogue (t) of the substance to be assayed bind to the labeled specific binding substance (s) in competition. Because of this, a portion of the labeled specific binding substance (s) (a portion bound to the analogue (t) of the substance to be assayed) does not bind to the immobilized specific binding substance (v) and is excluded from the assay system. Degree of such an exclusion is controlled by the quantitative ratio among the labeled specific binding substance (s), the analogue (t) of the substance to be assayed and the immobilized specific binding substance (v) and/or by the binding affinities of the substance (a) to be assayed and the analogue (t) of the substance to be assayed for the labeled specific binding substance (s). As the results, a dilution effect is obtained similar to the case of the process 1, and measurement at a proper signal intensity can be attained at a desired detection sensitivity.

In the practice of this process, it is preferable to use the immobilized specific binding substance (v) in large excess of the amount of the substance (a) to be assayed.

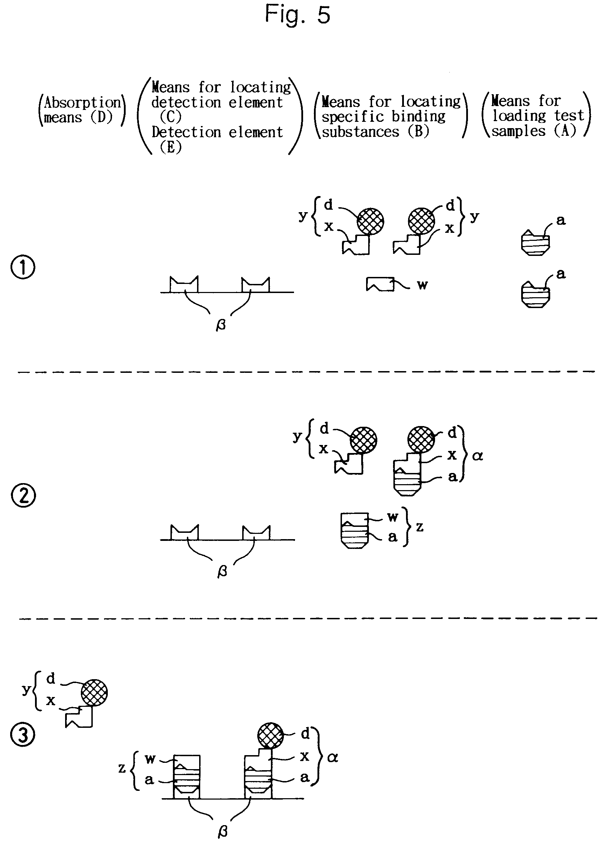

The example shown in Fig. 5 is an assay system in which the specified substance is a specific binding substance (w) which has a specific affinity for the substance (a) to be assayed, and the labeled material is a labeled specific binding substance (y) which is a compound resulting from binding of a labeling substance (d) to a specific binding substance (x) that has a specific affinity for the substance (a) to be assayed and interferes with the binding reaction of the substance (a) to be assayed with the specific binding substance (w).

In this case, the assay system is effected by a process which comprises the steps of:

allowing the substance (a) in a test sample to be assayed to undergo binding reaction simultaneously or successively with the specific binding substance (w) and with the labeled specific binding substance (y) which interferes with the specific binding substance (w) at the time of binding reaction with the substance (a) to be assayed;

allowing a binding substance (z) which is a compound resulting from the above binding reaction of the substance (a) to be assayed with the specific binding substance (w) and a binding substance ($\alpha$) which is a compound resulting from the above binding reaction of the substance (a) to be assayed with the labeled specific binding substance (y) to undergo binding reaction with an immobilized specific binding substance ($\beta$) that does not interfere with the specific binding substance (w) or the labeled specific binding substance (y) at the time of binding reaction with the substance (a) to be assayed, thereby immobilizing the binding substances (z) and ($\alpha$); and

reading off signals originated from the labeling substance (d) in the immobilized binding substance ($\alpha$).

This assay process will be called "process 5" hereinafter.

Typical examples of the binding conditions of each substance related to the assay system of the process 5 are as shown in Fig. 5.

In the process 5, the specific binding substance (w) and the labeled specific binding substance (y) bind to the substance (a) to be assayed in competition. Because of this, a portion of the labeled specific binding substance (y) cannot bind to the substance (a) to be assayed, and such an unbound portion of the labeled specific binding substance (y) is excluded from the assay system. Degree of such an exclusion is controlled mainly by the quantitative ratio of the labeled specific binding substance (y) to the specific binding substance (w) and/or by their binding affinities for the substance (a) to be assayed and the analogue (q). As the results, a dilution effect is obtained similar to the case of the process 1, and measurement at a proper signal intensity can be attained at a desired detection sensitivity.

In each of the illustrative examples (processes 1 to 5) described above, a compound resulting from binding of a labeling substance to a specific binding substance which has a specific affinity for a substance to be assayed is used as the labeled material.

The following describes another type of examples in which the labeled material is a compound resulting from binding of a labeling substance to a substance which binds to a specific binding substance having a specific affinity for a substance to be assayed and does not interfer with the binding reaction of the substance to be assayed with the specific binding substance.

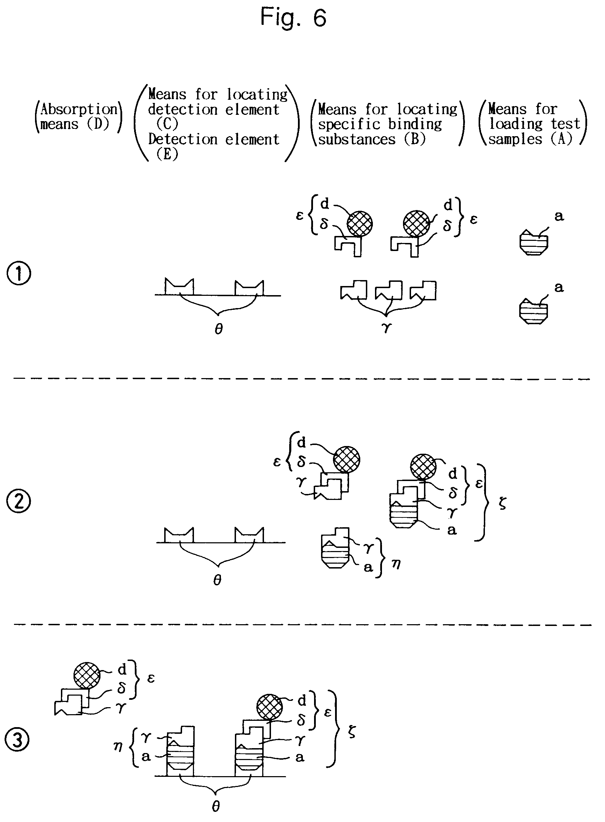

The example shown in Fig. 6 is an assay system in which the specified substance is a specific binding substance ($\gamma$) which has a specific affinity for the substance (a) to be assayed, and the labeled material is a labeled specific binding substance ($\epsilon$) which is a compound resulting from binding reaction of the labeling substance (d) with a specific binding substance ($\delta$) that has a specific affinity for the specific binding substance ($\gamma$) and does not interfer with the binding of the substance (a) to be assayed to the specific binding substance ($\gamma$).

In this case, the assay system is effected by a process which comprises the steps of:

allowing the specific binding substance ($\gamma$) having a specific affinity for the substance (a) to be assayed to undergo binding reaction simultaneously or successively with the substance (a) in a test sample to be assayed and with the labeled specific binding substance ($\epsilon$) which does not interfer with the substance (a) to be assayed at the time of binding reaction with the specific binding substance ($\gamma$);

allowing a binding substance ($\zeta$) which is a combined material resulting from the above binding reaction of the substance (a) to be assayed with the specific binding substance ($\gamma$) and the labeled specific binding substance ($\epsilon$) and a binding substance ($\eta$) which is a combined material resulting from the above binding reaction of the substance (a) to be assayed with the specific binding substance ($\gamma$) to undergo binding reaction with an immobilized specific binding substance ($\theta$) that has a specific affinity for the substance (a) to be assayed and is not interfered with the binding reaction of the substance (a) to be assayed with the specific binding substance ($\gamma$), thereby immobilizing said binding substances ($\zeta$) and ($\eta$); and

reading off signals originated from the labeling substance (d) in the immobilized binding substance ($\zeta$).

This assay process will be called "process 6" hereinafter.

Typical examples of the binding conditions of each substance related to the assay system of the process 6 are as shown in Fig. 6.

In the process 6, a portion of the labeled specific binding substance ($\epsilon$) binds to the specific binding substance (γ) and is excluded from the assay system when the amount of the specific binding substance (γ) is equal to or higher than the amount of the labeled specific binding substance (ε). Degree of such an exclusion is controlled mainly by the quantitative ratio of the specific binding substance (γ) to the labeled specific binding substance (ε). As the results, a dilution effect is obtained similar to the case of the process 1, and measurement at a proper signal intensity can be attained at a desired detection sensitivity.

In the illustrative examples described above (processes 1 to 6), the principle of competitive binding is not applied to the immobilized material. The processes 1 to 6 are characterized in that, though signals originated from a labeling substance increase as the amount of a substance to be assayed is increased, increasing rate of the signals is low in comparison with a process having no dilution effect, while maximum intensity of the signals is almost the same as that of the process having no dilution effect.

As has been described in the foregoing using Fig. 10 (I) as a reference, the prior art process for the reduction of detection sensitivity entails decrease in the maximum intensity of signals. On the contrary, assay results can be judged more accurately by the use of the process of the present invention (aforementioned processes 1 to 6 for example) because, as shown in Fig. 10 (II), signal intensity can be set easily to the proper level M when the amount of a substance to be assayed (for qualitative judgement for instance) is at a boundary level (N1, N2, N3 or N4) and, when the proper signal intensity is set to the level M, maximum intensity of signals hardly decreases in comparison with a case in which the dilution effect of the specified substance cannot be obtained (curve 1). In the figure, curves 2 to 4 belong to the present invention.

The following describes illustrative examples of the specific binding assay process of the second aspect of the present invention in which substances are bound to an immobilized material by competitive binding.

According to the second aspect of the present invention, there is provided a process for specific binding assay in which a substance in a test sample to be assayed is detected qualitatively or quantitatively making use of specific binding reaction, which comprises including a specified substance in an assay system and, by the effect of the presence of said specified substance, increasing amounts of a labeled material to be measured as an index of the amount of said substance to be assayed.

The specified substance and the labeled material are the same as those defined in the foregoing.

The following describes illustrative examples of the second aspect of the present invention based on figures attached hereto. These illustrative examples are characterized in that, though signals originated from a labeling substance decrease as the amount of a substance to be assayed is increased, decreasing rate of the signals is low in comparison with a process having no dilution effect, while maximum intensity of the signals is almost the same as that of the process having no dilution effect.

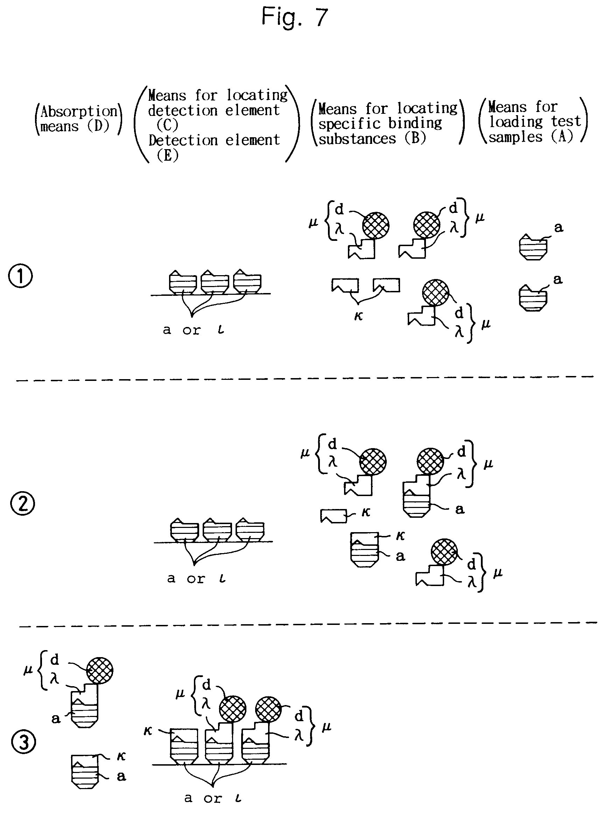

The example shown in Fig. 7 is an assay system in which the specified substance is a specific binding substance (x) having a specific affinity for the substance (a) to be assayed, and the labeled material is a labeled specific binding substance (μ) which is a compound resulting from binding of the labeling substance (d) to a specific binding substance (λ) that has a specific affinity for the substance (a) to be assayed and interferes with the binding reaction of the substance (a) to be assayed with the specific binding substance (x).

In this case, the assay system is effected by a process which comprises the steps of:
allowing the specific binding substance (x) having specific affinities for the substance (a) to be assayed and an analogue ( l) thereof and the labeled specific binding substance (μ) which interferes with the specific binding substance (x) at the time of the binding reaction with the substance (a) to be assayed and said analogue ( l) to undergo binding reaction simultaneously or successively with the substance (a) in a test sample to be assayed and with immobilized form of the substance (a) to be assayed or of the analogue ( l), thereby immobilizing at least a portion of the labeled specific binding substance (μ) on the immobilized substance (a) to be assayed or the immobilized analogue ( l); and
reading off signals originated from the labeling substance (d) in the immobilized labeled specific binding substance (μ).

This assay process will be called "process 7" hereinafter.

When the analogue ( l) of the substance to be assayed is used as the immobilized material, it may be any type of analogies, provided that the substance (a) to be assayed and the analogue ( l) interfere with each other in their binding to the sites related to one specific binding reaction in the specific binding substance (x) and the labeled specific binding substance (μ).

Typical examples of the binding conditions of each substance related to the assay system of the process 7 are as shown in Fig. 7.

In the process 7, the specific binding substance (x) and the labeled specific binding substance (μ) bind in competitive manner to the substance (a) in a test sample to be assayed and the immobilized analogue ( l) or the immobilized substance (a) to be assayed. As the results, the labeled specific binding substance

($\mu$) is excluded from the assay system when it binds to the substance (a) in the test sample to be assayed. Because of the presence of a combined product of the specific binding substance ($x$) with the substance (a) in the test sample to be assayed, the amount of the excluded substance is small in comparison with an assay system in which the substance (a) in a test sample to be assayed and the immobilized analogue ($\iota$) or the immobilized substance (a) to be assayed bind in competitive manner to the labeled specific binding substance ($\mu$). In consequence, the amount of the labeled specific binding substance ($\mu$) which binds to the immobilized analogue ($\iota$) or the immobilized substance (a) to be assayed becomes large in comparison with an assay system having no dilution effect. In this way, the dilution effect is obtained with a proper signal intensity at a desired detection sensitivity.

In this assay system, a dual competitive binding reaction is effected, and the degree of the dilution effect is controlled by the quantitative ratio of the specific binding substance ($x$) to the labeled specific binding substance ($\mu$) and/or by the binding affinities of the specific binding substance ($x$) and the labeled specific binding substance ($\mu$) for the analogue ($\iota$) and the substance (a) to be assayed.

In the practice of the process 7, it is preferable to use the immobilized analogue ($\iota$) or the immobilized substance (a) to be assayed in large excess of the amount of the specific binding substance ($x$).

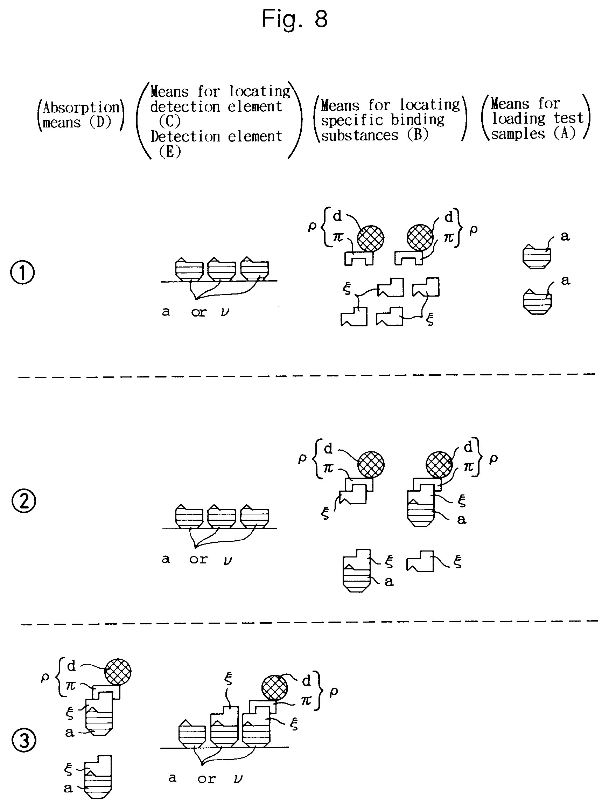

The example shown in Fig. 8 is an assay system in which the specified substance is a specific binding substance ($\varsigma$) having specific affinities for the substance (a) to be assayed and an analogue ($\nu$) of the substance to be assayed, and the labeled material is a labeled specific binding substance ($\rho$) which is a compound resulting from binding of the labeling substance (d) to a specific binding substance ($\pi$) that has a specific affinity for the specific binding substance ($\varsigma$) but does not interfere with the binding reaction of the substance (a) to be assayed with the specific binding substance ($\varsigma$).

In this case, the assay system is effected by a process which comprises the steps of:

allowing the specific binding substance ($\varsigma$) having specific affinities for the substance (a) to be assayed and the analogue ($\nu$) thereof to undergo binding reaction simultaneously or successively with the substance (a) to be assayed in a test sample, with immobilized form of the substance (a) to be assayed or of the analogue ($\nu$) and with the labeled specific binding substance ($\rho$) which does not interfere with the substance (a) to be assayed and the analogue ($\nu$) at the time of the binding reaction with the specific binding substance ($\varsigma$), thereby immobilizing at least a portion of the labeled specific binding substance ($\rho$) via the specific binding substance ($\varsigma$) on the immobilized substance (a) to be assayed or the immobilized analogue ($\nu$); and

reading off signals originated from the labeling substance (d) in the immobilized labeled specific binding substance ($\rho$).

This assay process will be called "process 8" hereinafter.

When the analogue ($\nu$) of the substance to be assayed is used as the immobilized material, it may be any type of analogies, provided that the substance (a) to be assayed and the analogue ($\nu$) interfere with each other in their binding to the sites related to one specific binding reaction in the specific binding substance ($\varsigma$).

Typical examples of the binding conditions of each substance related to the assay system of the process 8 are as shown in Fig. 8.

In the process 8, the substance (a) in a test sample to be assayed and the immobilized analogue ($\nu$) or the immobilized substance (a) to be assayed bind in competitive manner to the specific binding substance ($\varsigma$). In this instance, the specific binding substance ($\varsigma$) exists in two conditions; one to which the labeled specific binding substance ($\rho$) is bound or to be bound and the other free from the binding. As the results, the amount of the the labeled specific binding substance ($\rho$) which is excluded from the assay system after its binding to the substance (a) to be assayed via the specific binding substance ($\varsigma$) becomes small as the ratio of the amount of the specific binding substance ($\varsigma$) to the amount of the labeled specific binding substance ($\rho$) is increased. In consequence, the amount of the labeled specific binding substance ($\rho$) which binds to the immobilized analogue ($\nu$) or the immobilized substance (a) to be assayed becomes large in comparison with an assay system having no dilution effect. In this way, the dilution effect is obtained with a proper signal intensity at a desired detection sensitivity.

In this assay system, the specific binding substance ($\varsigma$) is used generally in a larger amount than the amount of the labeled specific binding substance ($\rho$), and the degree of the dilution effect is controlled by the quantitative ratio of the specific binding substance ($\varsigma$) to the labeled specific binding substance ($\rho$) and/or by the binding affinity of the specific binding substance ($\varsigma$) for the analogue ($\nu$) of the substance to be assayed.

In the practice of the process 8, it is preferable to use the immobilized analogue ($\nu$) or the immobilized substance (a) to be assayed in large excess of the amount of the specific binding substance ($\varsigma$).

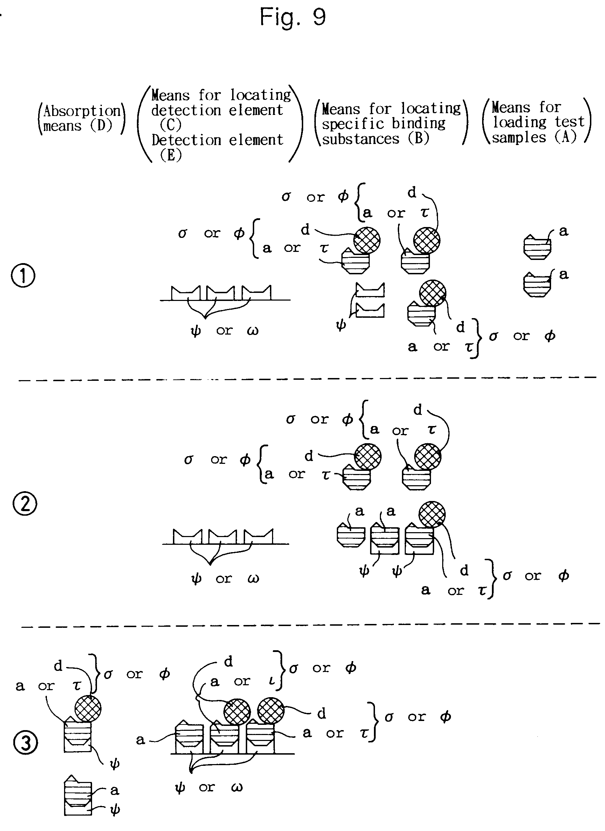

The example shown in Fig. 9 is an assay system in which the specified substance is a specific binding substance (φ) having a specific affinity for the substance (a) to be assayed, and the labeled material is a labeled substance (σ) to be assayed which is a compound resulting from binding of the labeling substance (d) to the substance (a) to be assayed or a labeled analogue (Φ) of the substance to be assayed which is a compound resulting from binding of the labeling substance (d) to an analogue (τ) of the substance to be assayed.

In this case, the assay system is effected by a process which comprises the steps of:

allowing the substance (a) in a test sample to be assayed and the labeled substance (σ) to be assayed or the labeled analogue (Φ) of the substance to be assayed to undergo binding reaction simultaneously or successively with the specific binding substance (φ) having specific affinities for the substance (a) to be assayed and the labeled analogue (Φ) of the substance to be assayed and with immobilized form of the specific binding substance (φ) or with an immobilized specific binding substance (ω) that interferes with the binding reaction of the specific binding substance (φ) with the substance (a) to be assayed and the labeled analogue (Φ) of the substance to be assayed, thereby immobilizing at least a portion of the labeled substance (σ) to be assayed or the labeled analogue (Φ) of the substance to be assayed; and reading off signals originated from the labeling substance (d) in the immobilized labeled substance (σ) to be assayed or the labeled analogue (Φ) of the substance to be assayed.

This assay process will be called "process 9" hereinafter.

In this instance, the specific binding substance (φ) and the specific binding substance (ω) interfere with each other in relation to the binding reaction of the substance (a) to be assayed with the labeled analogue (Φ) of the substance to be assayed. For example, when the substance (a) to be assayed is a substance which functions as an antigen and the labeled analogue (Φ) of the substance to be assayed is a compound formed by binding of the labeling substance (d) to an analogue that has the same antigenic determinant of the substance (a) to be assayed, these specific binding substances may be two different types of antibodies but having the same binding affinity for the antigenic determinant, or they may be two different types having specific affinities for different antigenic determinants but having such a relationship that the specific binding substance (ω) cannot bind to the substance (a) to be assayed or to the labeled analogue (Φ) of the substance to be assayed when the specific binding substance (φ) binds to the substance (a) or the analogue (Φ) in advance.

Typical examples of the binding conditions of each substance related to the assay system of the process 9 are as shown in Fig. 9.

In the process 9, the substance (a) in a test sample to be assayed and the labeled substance (σ) to be assayed or the labeled analogue (Φ) of the substance to be assayed bind in competitive manner to the soluble specific binding substance (φ) and the immobilized specific binding substance (φ) or the immobilized specific binding substance (ω). As the results, the labeled substance (σ) to be assayed or the labeled analogue (Φ) of the substance to be assayed to which the soluble specific binding substance (φ) is bound is excluded from the assay system. Because of the presence of a combined product of the soluble specific binding substance (φ) with the substance (a) in the test sample to be assayed, the amount of the excluded substance is small in comparison with an assay system in which the substance (a) in a test sample to be assayed and the labeled substance (σ) to be assayed or the labeled analogue (Φ) of the substance to be assayed bind in direct competitive manner to the immobilized specific binding substance (φ) or the immobilized specific binding substance (ω). In consequence, the amount of the labeled substance (σ) to be assayed or the labeled analogue (Φ) of the substance to be assayed which binds to the immobilized specific binding substance (φ) or the immobilized specific binding substance (ω) becomes large in comparison with an assay system having no dilution effect. In this way, the dilution effect is obtained with a proper signal intensity at a desired detection sensitivity.

In this assay system, a dual competitive binding reaction is effected, and the degree of the dilution effect is controlled by the quantitative ratio of the soluble specific binding substance (φ) to the immobilized specific binding substance (φ) or the immobilized specific binding substance (ω) and/or by the binding affinities of the specific binding substance (φ) and the specific binding substance (ω) for the substance (a) to be assayed and the labeled analogue (Φ) of the substance to be assayed.

In the illustrative examples described above (processes 7, 8 and 9), substances are bound to an immobilized material in competitive manner. By employing these processes, detection sensitivity can be decreased properly within a broad range in comparison with the prior art process to which the principle of competitive binding is applied.

That is, as has been described in the foregoing and as shown in Fig. 10 (III), the prior art competitive assay process for the reduction of detection sensitivity entails increase in the maximum intensity of signals. On the contrary, assay results can be judged more accurately by the use of the process of the present invention (aforementioned processes 7, 8 and 9 for example) because, as shown in Fig. 10 (IV), signal intensity can be set easily to a proper level when the amount of a substance to be assayed is at a desired level and, when the signal intensity is set to such a proper level, maximum intensity of signals hardly increases in comparison with a case in which the dilution effect of the specified substance cannot be obtained (curve 1). In the figure, curves 2 and 3 belong to the present invention.

Thus, the specific binding assay processes of the first and second aspects of the present invention have been described. In these processes, B/F separation and reading of signals originated from a labeling substance may be effected by known techniques.

Since illustrative examples of the specific binding assay process of the present invention include various modes, proper specified substance and process can be selected for instance from the processes 1 to 9, depending on the characteristics of the substance (a) to be assayed and specificities and affinities of specific binding substances to be used.

The following describes an assay system in which one of the two specific binding substances having specific affinity for the substance (a) to be assayed is immobilized and the other is labeled, using the processes 1 and 5 as examples.

When the specificity for the substance (a) to be assayed is higher in an immobilized specific binding substance than in a labeled specific binding substance, the process 1 may be useful in which the immobilized specific binding substance (b) is used as the specified substance.

On the other hand, when the specificity for the substance (a) to be assayed is higher in a labeled specific binding substance than in an immobilized specific binding substance, the process 5 may be useful in which the specific binding substance (w) is used as the specified substance.

Especially, when a test sample contains an additional substance which has structural similarity to the substance (a) to be assayed, specific control of the signal intensity of the substance (a) of interest can be attained for the first time by properly selecting an immobilized substance, a labeled material and a specified substance.

For example, according to the present invention, the amount of hCG in a test sample can be measured quantitatively without detecting LH or FSH contained in the sample which has structural similarity to hCG. When such an assay is carried out employing the process 1 or 5, specified substances and the like to be used may be as follows.

When anti-hCG$\beta$ antibody and anti-hCG$\alpha$ antibody are used as an immobilized substance and a labeled material, the specificity for hCG is higher in the anti-hCG$\beta$ antibody than in the anti-hCG$\alpha$ antibody. Because of this, when the anti-hCG$\beta$ antibody is used as immobilized antibody and the anti-hCG$\alpha$ antibody is used as antibody to be labeled, it is desirable to employ the process 1 in which the anti-hCG$\beta$ antibody functions as the specified substance. On the other hand, when the anti-hCG$\alpha$ antibody is used as immobilized antibody and the anti-hCG$\beta$ antibody is used as antibody to be labeled, it is desirable to employ the process 5 in which the anti-hCG$\beta$ antibody functions as the specified substance.

Though hCG can be measured by either of the processes 1 and 5, it is desirable to employ the process 1 in the case of the measurement of hCG because, in general, it is desirable to immobilize a specific binding substance having higher specificity for the substance (a) to be assayed when one of the two specific binding substances has a higher specificity for the substance (a) to be assayed and the other specific binding substance has a possibility of undergoing reaction with other substance than the substance (a) to be assayed both contained in a test sample.

Similarly, in the case of a CEA assay system in which antibody having specific reactivity with the CEA-specific epitopes is used in addition to other antibody which can react not only with CEA but also with other CEA-related antigens, it is desirable to use the anti-CEA antibody having higher specificity for CEA as the immobilized specific binding substance, the other antibody which can react also with CEA-related antigens as the labeled specific binding substance and a substance equivalent to the immobilized anti-CEA antibody as the specified substance.

In the case of the measurement of IgG antibody titers against particular virus, in general, virus-specific antigen is used as the immobilized specific binding substance and anti-human IgG antibody as the labeled specific binding substance. In such a case, it is desirable to employ the process 1 in which a substance equivalent to the immobilized virus-specific antigen is used as the specified substance, because the anti-human IgG antibody binds also to other human IgG than the anti-virus antibody.

The following describes the specific binding assay device of the third aspect of the present invention suitable for use in the practice of the assay processes of the first and second aspects of the invention.

The specific binding assay device of the third aspect of the present invention comprises at least one means (A) for loading a test sample, at least one means (B) for locating specific binding substances, at least one means (C) for locating a detecting element and at least one means (D) for absorbing liquid materials in that order, in which the means (B) contains the aforementioned specific binding substances and the means (C) contains a detecting element (E) where a certain substance is immobilized for use in indirect fixation of a labeling substance (d) which is used as an index of the amount or concentration of a substance (a) to be assayed.

By the use of such a device of the third aspect of the present invention, the substance (a) to be assayed flowed into the means (B) together with other substances in a test sample can be reacted with a specified substance, a labeled material and the like in appropriate orders by simply adding the test sample to the means (A), and the resulting sample containing reaction products (bound substances) and the like can be transferred into the means (C) in which necessary materials are fixed to the detecting element (E) and from which unnecessary materials are transferred into the absorbing means (D), all of these steps requiring no artificial handling.

The following describes the third aspect of the present invention based on the figures attached hereto.

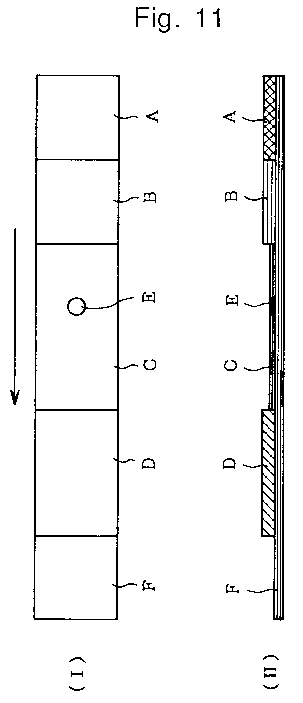

Fig. 11 illustrates a typical example of the assay device of the third aspect of the present invention, that is, a chromatography type specific binding assay device which comprises the means (A) for loading a test sample, the means (B) for locating specific binding substances, the means (C) for locating a detecting element and the means (D) for absorbing liquid materials in that order. In this instance, (I) and (II) are a plan view and a side view of the device, the detecting element (E) is located inside the means (C) and (F) is a supporting material.

Such an assay device may be constructed by closely arranging materials which constitute the means (A) for loading a test sample, the means (B) for locating specific binding substances, the means (C) for locating a detecting element and the means (D) for absorbing liquid materials on the supporting material (F) in such a manner that a liquid material can flow through each material uniformly by capillary action. In this instance, the supporting material (F) may be selected from a plastic plate, a plastic film and the like, preferably a plastic plate or the like to which a pressure sensitive adhesive double coated tape has been applied.

Since the means (A) is just a part where a test sample is loaded, the means (B) for locating specific binding substances may hold the purpose of the means (A).

However, when the means (A) is arranged as an independent part, the following advantage can be attained. That is, when the means (A) is used as an independent part, it functions as a pre-treatment site of a test sample for the transfer of only desired substances in the sample into the means (B) for locating specific binding substances and the means (C) for locating a detecting element, or as a site where rate, uniformity and the like of liquid flow is controlled, thereby rendering possible proper dissolution and binding reactions of specific binding substances (specified substance, labeled material and the like) in the means (B) and proper binding reaction on the detecting element (E).

According to the present invention, accuracy of the dilution effect decreases in some cases when dissolution and binding reactions of specific binding substances (specified substance, labeled material and the like) in the means (B) are not carried out properly.

Examples of the cause of such inappropriateness include: (1) inhibition of capillary action by particles contained in a test sample (for instance, non-specific aggregates of biological components, mucus components, blood cell components, microorganisms, incidentally contaminated dust and the like) which causes retention of liquid flow, decrease or increase in liquid flow rate and disorder of liquid flow front line; (2) fluctuation of the amount or properties (viscosity and the like) of a test sample added to the sample-loading means (A), which causes changes in the liquid flow rate; and (3) fluctuation of the amount of a test sample distributed to each unit in a specific binding assay device having a plurality of units which will be described later, that causes difference in the liquid flow rate among these units.

When minimization of non-specific adsorption of the substance (a) to be assayed in a test sample to a material of the sample-loading means (A) and stable absorption of liquid components by the material are taken into consideration, cellulose filter paper, glass fiber filter paper, cloth, non-woven fablic, porous ceramics or the like may be used as the material of the sample-loading means (A). Of these, cellulose filter paper for chromatography use may be used preferably because of its uniform paper quality, uniform water absorption rate and appropriate orientation of cellulose fibers.

Among filter paper articles for chromatography use, an article having relatively high water absorption rate, such as No. 526 (available from Advantech Toyo), may be used most preferably because of its uniform thickness which enables reproducible correspondence of the means (A) for loading a test sample to the amount of a test sample by defining the cutting size of the filter paper.

When the filter paper is cut into a square piece to use as the means (A) for loading a test sample, it is preferable to match its fiber orientation with the liquid flow direction and to keep one of its cut edges vertical to the liquid flow direction in contact evenly with a cut edge of the means (B) for locating specific binding substances, if necessary making an overlapping portion of 0.5 to 2.0 mm in the liquid flow direction.

14

In this way, uniform liquid flow can be maintained and the aforementioned problems (1) to (3) can be solved.

In the case of an assay device having a plurality of units in which distribution of liquid components in a test sample into the means (B) for locating specific binding substances is required, the aforementioned problem (3) can be solved when the (A) for loading a test sample is formed by superposing a square piece of the filter paper on another piece or each of a plurality of pieces which are in contact with a plurality of the means (B), in such a manner that orientation of the piled pieces becomes vertical.

As the sample-loading means (A), the filter paper piece may be used as it is or as a dried piece after impregnating it with various additive agents.

For example, surface active agents are effective in solving the aforementioned problems (1) to (3). Such effects can be obtained most properly when a nonionic surface active agent, such as a 0.01 to 0.5% solution of Tween 20 or Triton X-100, is added to filter paper or the like to a maximum absorption capacity of the material and then the soaked material is dried.

Also, protein components are effective in suppressing changes in the properties of liquid components in a loaded test sample. For example, such an effect can be obtained most properly when 0.05 to 0.5% of bovine serum albumin or gelatin dissolved in 0.067 M phosphate buffer (pH 5.5 to 7.5) or in 0.1 M Tris buffer (pH 6.0 to 8.0) is added to filter paper or the like to a maximum absorption capacity of the material and then the soaked material is dried.

These techniques effected by the material quality, communicability or additive agents in the sample-loading means (A) are in no way to be taken as limiting in relation to the control of reactions and the like in and after the means (B) for locating specific binding substances. These effects can be improved further by combining characteristics of the means (A) with the means (B) and/or the means (C) for locating a detecting element. Effects of such combination techniques will be described later.

Similar to the case of the sample-loading means (A), the means (B) for locating specific binding substances may be prepared preferably from a material which has a uniform quality, can be processed into a strict size and shows minimum non-specific adsorption of the substance (a) to be assayed which is contained in a test sample and of the components to be located in the means (B) such as specific binding substances, a substance to be assayed, an analogue of the substance to be assayed, a labeled material and the like. For example, cellulose filter paper, glass fiber filter paper, cloth, non-woven fabric, porous ceramics or the like may be used preferably as the material of the means (B). The means (B) may be prepared by drying one of the above materials, especially glass fiber filter paper or polyester-based non-woven fabric, which has been impregnated with proper amounts of specific binding substances, a substance to be assayed, an analogue of the substance to be assayed, a labeled material and the like together with additive agents such as a non-specific adsorption preventing component, a stabilizing component, a dissolution controlling component and the like.

It is obvious that trapping of a specified substance, a labeled material, a substance to be assayed and the like on the material of the means (B) for locating specific binding substances caused by their non-specific adsorption will result in poor measuring accuracy. Even if they are not trapped, the material of the means (B) shows different week mutual reaction with the specified substance, the labeled material or the substance to be assayed. Because of this, difference in the travel speed occurs between the labeled material and the specified substance during their passage through the material of the means (B), which sometimes causes difficulty in controlling the dilution effect and entails poor accuracy of detection sensitivity setting. For example, when the specified substance has a higher travel speed than that of the labeled material, effect of the specified substance increases and the signal intensity becomes too low in the case of the aforementioned assay processes 3, 5 and 6.

In order to prevent such phenomena by minimizing differences in the travel speed among a substance to be assayed, a labeled material, a specific binding substance and the like, it is desirable to use the aforementioned additive agents, especially a non-specific adsorption preventing component. For example, a proper means (B) for locating specific binding substances may be obtained making use of a material which is prepared by impregnating an appropriate material with a protein component and/or a surface active agent to a maximum absorption capacity of the material and then drying the soaked material directly or after washing. In this instance, a 0.1 to 1% solution of bovine serum albumin or gelatin may be used preferably as the protein component, and a nonionic surface active agent such as a 0.01 to 0.5% solution of Tween 20 or Triton X-100 may be used preferably as the surface active agent.

In the case of the assay device of the present invention, activities of a specified substance, a labeled material and the like included in the means (B) for locating specific binding substances sometimes decrease during storage of the device. When the decreasing rate of the activities of these substances differs from one another, the detection sensitivity set at the time of the production of the device cannot be

15

reproduced. In order to solve such a problem, it is desirable to prepare the means (B) making use of a material which has been dried after its impregnation with a sugar solution as a stabilizing agent, especially a 0.5 to 8% solution of a saccharose or lactose, to a maximum absorption capacity of the material.

In addition, in the case of the assay device of the present invention, difference in the dissolution rate of the specified substance, labeled material and the like included in the means (B) may also exert influence on the detection sensitivity control. Such a difference in the dissolution rate depends on the properties of a test sample and types and amounts of the specified substance, labeled material and the like, and the degree of such a difference differs depending on the specified substance-aided controlling method of the amount of the labeled material which is measured as an index of the amount of a substance to be assayed.

Such a problem caused by different dissolution rates may be solved in general when the means (B) is prepared using a dissolution controlling component, especially a 0.5 to 8% solution of a saccharose. When the labeling substance (d) is a highly hydrophobic substance such as a dye, surface active agents, especially a nonionic surface active agent, may be used to increase its dissolution rate. However, when the labeling substance (d) is bound to a specific binding substance and the like by means of non-covalent binding, the dissolution controlling component, especially a surface active agent, functions as a destabilizing component during storage of the device, thus entailing undesirable dissociation of the labeling substance (d) from a specific binding substance and the like. In such a case, similar effect on the solubility improvement of various substances in the means (B) for locating specific binding substances can be obtained by including the dissolution controlling component in the sample-loading means (A) instead of including it in the means (B).

Constitution of the means (C) for locating a detecting element is divided into two types depending on the immobilization method of substances.

That is, when a certain substance is immobilized on a part of a means (C)-constituting material by a physical or chemical binding method, the immobilized part becomes the detecting element (E) (direct immobilization). On the other hand, when a certain substance is immobilized on fine particles by a physical or chemical binding method and the resulting particles are trapped in a means (C)-constituting porous material, another type of the means (C) in which the fine particles (detecting element (E)) are trapped is formed (indirect immobilization).

Preferred examples of the means (C)-constituting material in the case of the direct immobilization include porous cellulose derivative film, porous nitrocellulose film, glass capillary, porous ceramics, glass fiber filter paper, non-woven fabric, cloth, plastic net and the like. If necessary, active groups for binding use may be added to these materials. In the case of the indirect immobilization, preferred examples of the means (C)-constituting material include: fine particles such as polystyrene fine particles, latex, glass powder and the like, or modified products thereof to which active groups for binding use are added; and glass fiber filter paper, non-woven fabric, cloth, plastic net, porous cellulose derivative film, porous nitrocellulose film, porous ceramics and the like (as materials for use in the trapping of these fine particles).

When the means (C) for locating a detecting element is prepared, various additive agents may be used. Roles and the like of these agents are the same as those described in the foregoing with regard to the means (B) for locating specific binding substances.

The means (B) and (C) may be made into one section, which will be described later in detail.

The means (D) for absorbing liquid materials is a part for use in the absorption and keeping of added liquid materials such as a test sample and, therefore, is composed of a water absorbing material such as non-woven fabric, woven fabric, plastic net or the like in which a water absorbing polymer is trapped or cellulose filter paper or the like.

The liquid-absorbing means (D) has a function to remove excess amounts of a labeled material and the like from the detecting element (E) by absorbing them through increased liquid flow rate, thereby improving measuring accuracy of the signal intensity. At the same time, however, changes in the liquid flow rate sometimes entail fluctuation of the signal intensity. In that case, a downstream portion of the detecting element (E) in the means (C) for locating a detecting element may be used as the liquid-absorbing means (D), instead of using it as a separate part.

Thus, each part of the assay device of the third aspect of the present invention has been described. Constitutions of these parts contribute to the regulation of the detection sensitivity through their mutual relationships.

The following further describes the assay device of the third aspect of the present invention from a viewpoint of the correlation of these parts.

According to the assay device of the third aspect of the present invention, flow rate of liquid components in a test sample into the means (B) for locating specific binding substances (liquid flow rate) can be set at will at the time of the production of the device by optionally combining material quality, size, thickness, additive agents and the like of the sample-loading means (A), material quality, size, thickness, additive agents and the like of the means (B) for locating specific binding substances and communicabilities between the means (A) and (B) such as contact line length, overlapped area, contact pressure and the like. By setting the liquid flow rate in this way, specific binding reactions in and after the means (B) can be controlled in such a manner that each reaction can be effected optimally depending on each reaction method.

In the same manner, flow rate of liquid components in a test sample into the means (C) for locating a detecting element (liquid flow rate) can be set at will by optionally combining material quality, size, thickness, additive agents and the like of the means (B) for locating specific binding substances, material quality, size, thickness, additive agents and the like of the means (C) for locating a detecting element and communicabilities between the means (B) and (C) such as overlapped area, contact pressure and the like. By setting the liquid flow rate in this way, reactions in the detecting element (E) can be controlled in such a manner that each reaction can be effected optimally depending on each reaction method.

Especially, signal intensity in the detecting element (E) is determined by the amount of a labeled material which accumulates in the detecting element (E) by a specific binding reaction, and the signal intensity generally increases as the amount of the element (E)-passing liquid increases. In addition, the signal intensity reaches a constant level when the liquid flow ceases. In consequence, in order to realize accurate control of the detection sensitivity in the presence of a specified substance, it is necessary to control the amount of the element (E)-passing liquid or termination of the liquid flow. According to the assay device of the third aspect of the present invention, the amount of liquid components in a test sample passing through the detecting element (E) can be set at will and termination of the liquid flow can be controlled accurately at will, by selecting and combining proper qualities, sizes, thicknesses and the like of constituting materials of the means (A) for loading a test sample, the means (B) for locating specific binding substances, the means (C) for locating a detecting element and the means (D) for absorbing liquid materials.

With regard to the constituting material of each means, the materials described in the foregoing may be used in a proper combination. When it is desirable to employ the same material quality in at least two of or all four of the means (A), (B), (C) and (D), one and the same material may be used or a region in common with 2 or 3 of the means may be arranged.

Next, the size of these means is described.

When the assay device of the third aspect of the present invention is used, its dilution effect, efficiency and accuracy are influenced by the size of each of the means (A) for loading a test sample, the means (B) for locating specific binding substances, the means (C) for locating a detecting element and the means (D) for absorbing liquid materials, even if qualities and additive agents in the constituting materials of these means are defined. The reason for this is that the signal intensity in the detecting element (E) depends on the size of each means due, mainly, to the following factors (1) and (2).

(1) Reaction time related to a controlling step in the detecting element (E), in which the signal intensity that corresponds to the amount or concentration of the substance (a) to be assayed in a test sample is controlled by the effect of a specified substance, depends on the rate and distance of the liquid flow, that is, mainly on the length and thickness of constituting materials in the liquid flow direction from the sample-loading means (A) to the detecting element (E), especially from the means (A)-side cut edge of the means (B) for locating specific binding substances where a specified substance is included to the detecting element (E).

(2) The signal intensity in the detecting element (E) depends on the amount of liquid components which pass through the element (E), and such an amount depends mainly on the length and thickness of the element (E) and its downstream portion in the means (C) for locating a detecting element and those of the means (D) for absorbing liquid materials.

In consequence, the size of the means (A) for loading a test sample may preferably be determined by taking maximum liquid-absorbing capacity of its constituting material into consideration. Especially, it is preferable to determine the size of the sample-loading means (A) in such a way that the maximum liquid-absorbing capacity of the constituting material becomes equivalent to 20 to 80% of the liquid components in a test sample to be loaded.

Illustratively, a preferred size of the sample-loading means (A) may be in the range of from 0.6 to 2.3 cm$^2$ when a cellulose filter paper No. 526 for chromatography use (available from Advantech Toyo) is used as the constituting material and a test sample is loaded in an amount of 200 $\mu$l.

Sizes of the means (B) for locating specific binding substances and the means (C) for locating a detecting element may be determined in relation to the size of the means (A) for loading a test sample.

For example, when the amount of a test sample to be loaded is set to 200 $\mu$l, the width of each means

17

is set to 10 mm and the sample-loading means (A) is prepared from the just described cellulose filter paper No. 526 for chromatography use having a size of 10 mm in length, 10 mm in width and 0.7 mm in thickness, the means (B) for locating specific binding substances may be prepared preferably from a polyester non-woven fabric having a length of from 2 to 50 mm and a thickness of from 0.1 to 2 mm or from a glass fiber filter paper having a length of from 2 to 50 mm and a thickness of from 0.1 to 2 mm, and the means (C) for locating a detecting element may be prepared preferably from a nitrocellulose film having a length of from 2 to 100 mm and a thickness of from 0.05 to 2 mm, provided that the aforementioned dependency of the signal intensity in the detecting element (E) on the size of the means (B) and (C) is not taken into consideration.

However, when the just described dependency is taken into consideration, it is necessary to optimize efficiency and accuracy of the signal intensity control in the detecting element (E). That is, in order to prevent decrease in the maximum intensity of signals which are measured as the index of the amount of the substance (a) to be assayed in a test sample, it is most preferable to set the length of the means (B) for locating specific binding substances to 10 mm or shorter when a polyester non-woven fabric having a thickness of for example 0.5 mm is used, and to set the length of the means (C) for locating a detecting element, from its means (B)-side cut edge to the detecting element (E), to 50 mm or shorter when a nitrocellulose film having a thickness of for example 0.16 mm is used.

In addition, the size of the means (B) for locating specific binding substances should be changed in response to the amount of specific binding substances (a specified substance, a labeled material and the like) to be included in the means (B), because location of a large quantity of substances in a small area under a dry condition decreases solubility of the substances. In consequence, it is necessary to select a proper area in response to the properties and amounts of specific binding substances to be located.

Next, communicability of the means is described.

According to the assay device of the third aspect of the present invention, when communication among these means is effected by connecting them through cut edges of their constituting materials having a constant sectional area, travel efficiency of liquid components increases as the contact line length increases and, as the results, liquid flow rate increases. Preferred contact line length may be in the range of from 3 to 20 mm in general, though it differs depending on the quality and thickness of the constituting material of each means.

The communicability exerts influence on the uniformity of the rate and front line of the liquid flow and, as the results, exerts influence on the signal intensity control. In consequence, it is desirable to effect communication among these means by uniform contact, more preferably by their connection with an overlapping portion of 0.5 to 2 mm.

As has been described in the foregoing, the means of the assay device of the third aspect of the present invention control the signal intensity through their mutual relation.

In the assay device of the third aspect of the present invention, substances to be adhered to or immobilized on the means (B) for locating specific binding substances and the means (C) for locating a detecting element may be obtained by any convenient method. For example, these substances may be purified articles or products obtained by means of genetic engineering techniques or, when they are antibodies, they may be monoclonal antibodies or polyclonal antibodies. In addition, a combined material of the labeling substance (d) with a certain substance may also be obtained by any convenient technique.

It is preferable to produce the assay device of the third aspect of the present invention by preparing each means, arranging each means thus prepared on a predetermined position and then applying a transparent pressure sensitive tape having a thickness of 100 $\mu$m or less to the thus arranged set of means with such an even pressure that capillary action is not damaged.

Thus, construction of the assay device of the third aspect of the present invention has been described. The following describes assay devices for use in the practice of the illustrative examples which have been described in the foregoing in relation to the first and second aspects of the present invention.

In a specific binding assay device for use in the practice of the aforementioned process 1, the specific binding substance (b) and the labeled specific binding substance (e) are included in the means (B) for locating specific binding substances, and the specific binding substance (b) or the specific binding substance (g) is immobilized to the detecting element (E).

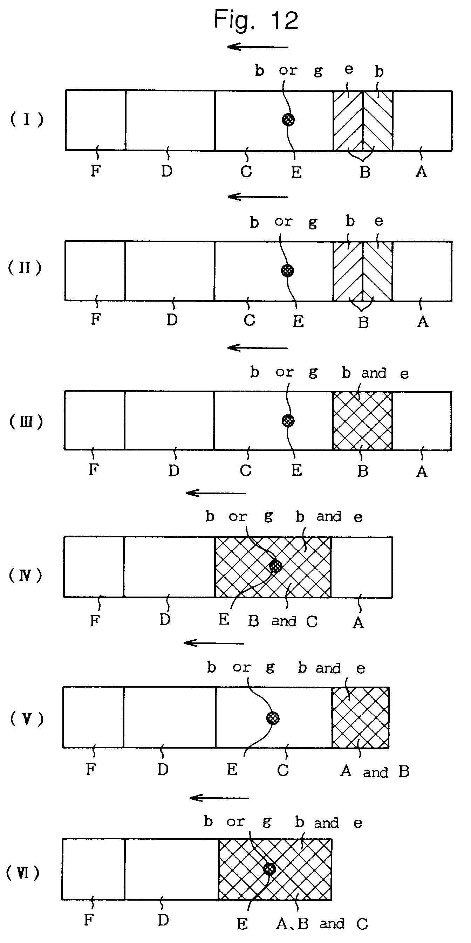

In this instance, the specific binding substance (b) and the labeled specific binding substance (e) included in the means (B) for locating specific binding substances may be arranged in order (see Fig. 12 I and II) along the flow direction of a test sample (a direction indicated with an arrow in Fig. 12 I) or in a mixed state (see Fig. 12 III). The means (B) for locating specific binding substances and the means (C) for locating a detecting element may be arranged on the same position (see Fig. 12 IV). In addition, a device in which the means (A) for loading a test sample and the means (B) for locating specific binding substances are arranged on the same position (see Fig. 12 V) and another device in which the means (A), (B) and (C) are arranged on the same position (see Fig. 12 VI) may also be included within the scope of the third aspect of the present invention.

In a specific binding assay device for use in the practice of the aforementioned process 2, the specific binding substance (i) and the labeled specific binding substance (k) are included in the means (B) for locating specific binding substances, and the specific binding substance (l) is immobilized to the detecting element (E).

In this instance, the specific binding substance (i) and the labeled specific binding substance (k) included in the means (B) for locating specific binding substance may be arranged in a mixed state. The means (B) for locating specific binding substances and the means (C) for locating a detecting element may be arranged on the same position where the specific binding substance (i), the labeled specific binding substance (k) and the immobilized specific binding substance (l) are arranged in a mixed state. Also, the means (A) for loading a test sample and the means (B) for locating specific binding substances may be arranged on the same position. In addition, the means (A), (B) and (C) may be arranged on the same position. Most preferably, the means (B) for locating specific binding substances may exist as an independent section in which the specific binding substance (i) and the labeled specific binding substance (k) are arranged in that order along the flow direction of a test sample.

In a specific binding assay device for use in the practice of the aforementioned process 3, the labeled specific binding substance (n) and the analogue (q) of a substance to be assayed are included in the means (B) for locating specific binding substances, and the specific binding substance (o) is immobilized to the detecting element (E).

In this instance, the labeled specific binding substance (n) and the analogue (q) of a substance to be assayed included in the means (B) for locating specific binding substance may be arranged in an optional order along the flow direction of a test sample. The means (B) for locating specific binding substances and the means (C) for locating a detecting element may be arranged on the same position. In addition, other variations described above in relation to the devices for use in the practice of the processes 1 and 2 may also be used.

In a specific binding assay device for use in the practice of the aforementioned process 4, the labeled specific binding substance (s) and the analogue (t) of a substance to be assayed are included in the means (B) for locating specific binding substances, and the specific binding substance (v) is immobilized to the detecting element (E).

In this instance, the labeled specific binding substance (s) and the analogue (t) of a substance to be assayed included in the means (B) for locating specific binding substance may be arranged in a mixed state. The means (B) for locating specific binding substances and the means (C) for locating a detecting element may be arranged on the same position. Other variations described above may also be used. Most preferably, the means (B) for locating specific binding substances may exist as an independent section in which the analogue (t) of a substance to be assayed and the labeled specific binding substance (s) are arranged in that order along the flow direction of a test sample.

In a specific binding assay device for use in the practice of the aforementioned process 5, the specific binding substance (w) and the labeled specific binding substance (y) are included in the means (B) for locating specific binding substances, and the specific binding substance ($\beta$) is immobilized to the detecting element (E).

In this instance, the specific binding substance (w) and the labeled specific binding substance (y) included in the means (B) for locating specific binding substance may be arranged in a mixed state. The means (B) for locating specific binding substances and the means (C) for locating a detecting element may be arranged on the same position. Other variations described above may also be used. Most preferably, the means (B) for locating specific binding substances may exist as an independent section in which the specific binding substance (w) and the labeled specific binding substance (y) are arranged in that order along the flow direction of a test sample.

In a specific binding assay device for use in the practice of the aforementioned process 6, the specific binding substance ($\gamma$) and the labeled specific binding substance ($\epsilon$) are included in the means (B) for locating specific binding substances, and the specific binding substance ($\theta$) is immobilized to the detecting element (E).

In this instance, the specific binding substance ($\gamma$) and the labeled specific binding substance ($\epsilon$) included in the means (B) for locating specific binding substance may be arranged in a mixed state or in an optional order along the flow direction of a test sample. Also, the means (B) for locating specific binding substances and the means (C) for locating a detecting element may be arranged on the same position. Other variations described above may also be used.

19

In a specific binding assay device for use in the practice of the aforementioned process 7, the specific binding substance ($x$) and the labeled specific binding substance ($\mu$) are included in the means (B) for locating specific binding substances, and the substance (a) to be assayed or the analogue ( $l$) of the substance to be assayed is immobilized to the detecting element (E).

In this instance, the specific binding substance ($x$) and the labeled specific binding substance ($\mu$) included in the means (B) for locating specific binding substance may be arranged in a mixed state or in order along the flow direction of a test sample. Also, the means (B) for locating specific binding substances and the means (C) for locating a detecting element may be arranged on the same position where the specific binding substance ($x$), the labeled specific binding substance ($\mu$) and the immobilized substance (a) to be assayed or the immobilized analogue ( $v$) of the substance to be assayed are located in a mixed state. Other variations described above may also be used.

In a specific binding assay device for use in the practice of the aforementioned process 8, the specific binding substance ($\varsigma$) and the labeled specific binding substance ($\rho$) are included in the means (B) for locating specific binding substances, and the substance (a) to be assayed or the analogue ($v$) of the substance to be assayed is immobilized to the detecting element (E).

In this instance, the specific binding substance ($\varsigma$) and the labeled specific binding substance ($\rho$) included in the means (B) for locating specific binding substance may be arranged in a mixed state or in order along the flow direction of a test sample. Also, the means (B) for locating specific binding substances and the means (C) for locating a detecting element may be arranged on the same position where the specific binding substance ($\varsigma$), the labeled specific binding substance ($\rho$) and the immobilized substance (a) to be assayed or the immobilized analogue ($v$) of the substance to be assayed are located in a mixed state. Other variations described above may also be used.

In a specific binding assay device for use in the practice of the aforementioned process 9, the labeled substance ($\sigma$) to be assayed or the labeled analogue ($\phi$) of the substance to be assayed and the specific binding substance ($\phi$) are included in the means (B) for locating specific binding substances, and the specific binding substance ($\phi$) or the specific binding substance ($\omega$) is immobilized to the detecting element (E).

In this instance, the labeled substance ($\sigma$) to be assayed or the labeled analogue ($\Phi$) of the substance to be assayed and the specific binding substance ($\phi$) included in the means (B) for locating specific binding substance may be arranged in a mixed state. Also, the means (B) for locating specific binding substances and the means (C) for locating a detecting element may be arranged on the same position. Other variations described above may also be used. Most preferably, the means (B) for locating specific binding substances may exist as an independent section in which the specific binding substance ($\phi$) and the labeled substance ($\sigma$) to be assayed or the labeled analogue ($\Phi$) of the substance to be assayed are arranged in that order along the flow direction of a test sample.

As a summary, Table 1 shows affinities of substances related to the specific binding reaction in each of the processes 1 to 9 which have been described based on Figs. 1 to 9. As shown in the table, when the assay device of the third aspect of the present invention is used for the practice of the processes 1 to 9, effects of the present invention vary in some cases depending on the arrangement of the labeled material and the specified substance in the means (B) for locating specific binding substances. For example, in the processes 2, 4, 5 and 9, it is preferable to arrange the specified substance on a position which is upstream of the labeled material and close to the means (A) for loading a test sample.

Serial arrangement of the specified substance and the labeled material in the means (B) for locating specific binding substances along the flow direction of a test sample may be effected, for example, by dividing the means (B) into two portions and including the specified substance and the labeled material separately in the divided portions, or by preparing a means (B-1) in which the specified substance is included and another means (B-2) containing the labeled material and then connecting them in an optional order.

## Table 1  Mutual affinity of substances related to the specific binding reaction

| | Substance *b | | | Affinity | | | | | |
|---|---|---|---|---|---|---|---|---|---|
| Pro-cess *a | A (label-ed) | B (speci-fied) | C (immobi-lized) | A and B | a and A | a and B | A and C | B and C | a and C |
| 1 | e | b | b or g | − | + | +*4 | − | − | + |
| 2 | k | i | l | − | + | + *3,4 | − | − | + |
| 3 | n | q | o | − | + | − | − | + | +*5 |
| 4 | s | t | v | +*1 | + | − | − | − | + |
| 5 | y | w | β | − | + | +*3 | − | − | + |
| 6 | ε | γ | θ | +*2 | − | + | − | − | + |
| 7 | μ | κ | a or ι | − | + | + | + | + | − |
| 8 | ρ | ξ | a or ν | +*2 | − | + | − | + | − |
| 9 | σ or φ | φ | φ or ω | +*1 | − | + | + | − | +*6 |

*a: Also indicates corresponding Figs. 1 to 9.

*b: A, labeled material; B, specified substance; C, immobilized substance

*1: Since foremost reaction of A with B is not desirable, it is preferable to arrange B to a position which is upstream of A and close to the sample-loading means.

*2: Foremost reaction of A with B is not undesirable.

*3: Since foremost binding of the substance (a) to A prior to the binding of (a) to B is not desirable, it is preferable to arrange B to a position which is upstream of A and close to the sample-loading means.

*4: Foremost binding of the substance (a) to A prior to the binding of (a) to B is not undesirable.

*5: Foremost binding reaction of B with C prior to the binding of (a) to C is not desirable, but such a phenomenon does not occur in general.

*6: Foremost binding reaction of A with C prior to the binding of (a) to C is not undesirable.

The assay device of the third aspect of the present invention is not restricted to the aforementioned single lane system. For example, in the case of the assay device for use in the practice of the process 1, there is provided a specific binding assay device capable of effecting semi-quantitative assay of a substance in a test sample, in which the means (A) for loading a test sample exists as a common section, the means (B) for locating specific binding substances and the means (C) for locating a detecting element exist as a plurality of independent units and the means (D) for absorbing liquid materials exists as a common section or is included in the units, wherein at least one of the specific binding substance (b) and the labeled specific binding substance (e) located in the means (B) and the specific binding substance (b) or the specific binding substance (g) located in the detecting element (E) is contained in each of the units in different amounts.

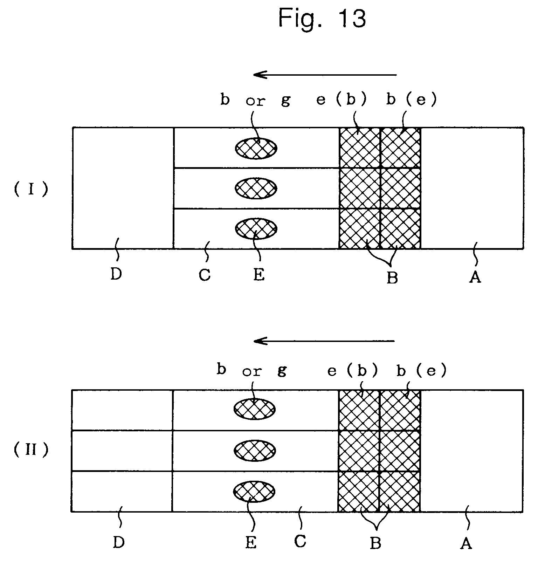

Illustrative examples of such a type of assay devices include a device shown in Fig. 13 (I) in which a plurality of units consisting of the means (B) for locating specific binding substances and the means (C) for locating a detecting element exist between the means (A) for loading a test sample and the means (D) for absorbing liquid materials, and another device shown in Fig. 13 (II) in which a plurality of units consisting of the means (B), (C) and (D) exist downstream side of the means (A) which is used as a common section.

In this instance, in Fig. 13 (I and II), the specific binding substance (b) and the labeled specific binding substance (e) located in the means (B) may be present in a mixed state.

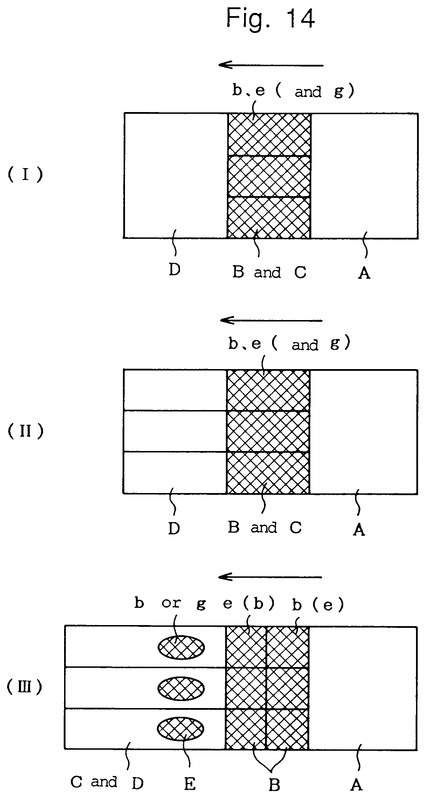

As a variation of the assay device shown in Fig. 13 (I), there is provided a device shown in Fig. 14 (I) in which a plurality of units, each of which consisting of the means (B) for locating specific binding substances and the means (C) for locating a detecting element, exist between the means (A) for loading a test sample and the means (D) for absorbing liquid materials. As a variation of the assay device shown in Fig. 13 (II), there is provided a device shown in Fig. 14 (II) in which a plurality of units, each unit consisting of the means (B) and (C), and a plurality of units of the means (D) exist downstream side of the means (A) which is used as a common section, or a device shown in Fig. 14 (III) in which a plurality of units of the means (B) and a plurality of another units, each unit consisting of the means (C) and (D), exist downstream side of the means (A) which is used as a common section.

In the case of the device shown in Fig. 14 (III), the means (D) for absorbing liquid materials is located on a downstream position of the detecting element (E) in the means (C) for locating a detecting element.

Other specific binding assay devices for use in the practice of the processes 2 to 9, which are capable of effecting semi-quantitative assay of a substance in a test sample, are not described herein, because they are basically the same as the just described devices for use in the practice of the process 1 except that the substances included in the means (B) for locating specific binding substances and the substance immobilized on the detecting element (E) are different from one another.

In these specific assay devices, amounts of adhered and/or immobilized substances vary depending on each unit and, as the results, the detection sensitivity in the detecting element (E) varies accordingly. Because of this, semi-quantitative assay can be effected without using a standard curve or a reference table. In this instance, the signal intensity can be controlled at the same level in each unit.

Any substance included in the device are quantitatively changeable. For example, in the case of the process 4, 7 or 8, it is preferable to use the substance immobilized on the detecting element (E) in excess amount, while amounts of one or all substances in the means (B) for locating specific binding substances are changed. When binding of a substance to an immobilized substance is effected by competitive manner (the process 7, 8 or 9 for instance), it is desirable to control the amount of a labeled material at a constant level. In this way, assay results at different sensitivity levels can be compared.

The assay device may have a unit which does not contain one of the unlabeled substances included in the means (B) for locating specific binding substances. In that case, such a unit does not show the dilution effect of the present invention, but comparison of assay results at different sensitivity levels can be attained by comparing results of this unit with those of other units which have the dilution effect.

When these devices are used for qualitative or semi-quantitative assay, it is desirable to use a visibly detectable labeling substance such as a dye, a pigment, a colored latex, a gold colloid or the like.

When used for quantitative assay, it is desirable to take a measure, such as establishment of partitions between units, to give independence to the means (C) for locating a detecting element in each unit, or to protect one unit from the influence of other means (C)-containing units.

Like the case of the aforementioned single lane devices, quality, size and thickness of the constituting material of each means, as well as mutual communicability among these means and the presence of additive agents, are important factors in these multiple unit devices. By varying these conditions in each unit, the detection sensitivity can be changed.

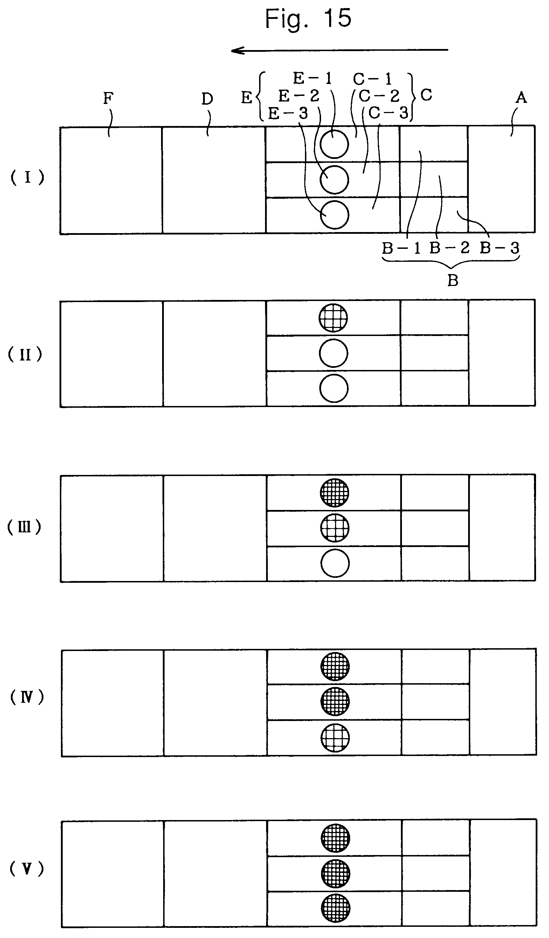

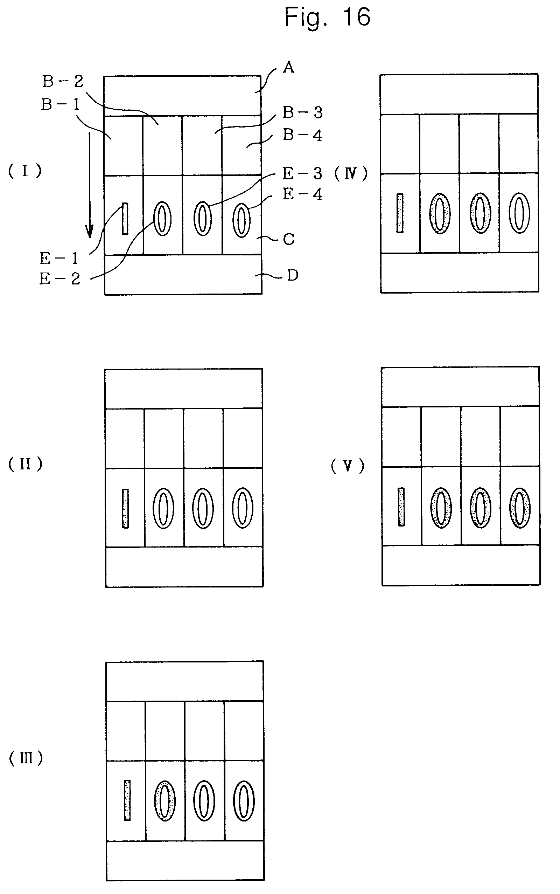

Examples of assays making use of such devices each having a plurality of units with different detection sensitivities are shown in Figs. 15 and 16.

In the assay device of Fig. 15, amounts or ratios of specific binding substances and the like to be included in three means for locating specific binding substances (B-1, B-2 and B-3) are changed, in order to vary detection sensitivities of three detecting elements (E-1, E-2 and E-3). In this instance, binding of substances to the immobilized substance is not effected in a competitive manner. In this figure, the illustration (I) is an example in which the amount or concentration of a substance to be assayed is less than the detection sensitivity, the illustration (II) is an example in which the amount or concentration of a substance to be assayed is equal to or larger than the detection sensitivity of E-1 and is less than the detection sensitivity of E-2, the illustration (III) is an example in which the amount or concentration of a substance to be assayed is equal to or larger than the detection sensitivity of E-2 and is less than the detection sensitivity of E-3 and the illustrations (IV) and (V) are examples in which the amount or the concentration of a substance to be assayed is equal to or larger than the detection sensitivity of E-3. When binding of substances to the immobilized substance is effected in a competitive manner, amounts or concentrations of a substance to be assayed become the reverse order of these illustrations.

The assay device shown in Fig. 16 is basically the same as that of Fig. 15, except that the results can be read off as numerical characters 1, 10, 100 and 1000.

These devices suitable for semi-quantitative assays are especially useful for the quick and easy semi-quantitative monitoring of, for example, a tumor marker after cancer operation, changes in the antibody titers in an infectious disease caused by virus or the like pathogen, concentration of a drug in blood, response on immunological rejection after transplantation, changes in the acute phase protein during inflammation, periodical changes in hormones and the like.

For example, human placental lactogen (hPL), a hormone produced in and secreted from human placenta, has a short half life of 15 minutes in blood. Because of this, it is considered that the blood hPL level just reflects placental functions.

Normal blood hPL level in the late stage of pregnancy is 4 to 10 $\mu$g/ml, and a concentration of 4 $\mu$g/ml or below is regarded as a critical level of causing imminent abortion and the like. Since the hPL level varies depending on the week of pregnancy, individual difference and the like, it is necessary to check blood hPL levels in each pregnant woman periodically. In consequence, great concern has been directed toward the development of a quick assay method which can correspond to urgent cases such as imminent abortion and the like through routine check of the blood hPL level as prenatal care.

When the blood hPL level is measured using a semi-quantitative assay device of the present invention having three detecting elements (E) with detection sensitivities of 2, 4 and 6 $\mu$g/ml, the hPL concentration in a sample can be divided into measuring ranges of <2 $\mu$g/ml, 2 to 4 $\mu$g/ml, 4 to 6 $\mu$g/ml and >6 $\mu$g/ml. As the results, normality and abnormality of the blood hPL level can be judged correctly taking individual differences and weeks of pregnancy into consideration.

A hemagglutination test or a latex agglutination test has been used as a simple quantitative assay of hPL. These prior art methods, however, require serial dilution of a test sample to obtain semi-quantitative data. According to the assay device of the present invention, semi-quantitative detection of hPL concentration in a test sample can be achieved without requiring such a serial dilution step, by simply adding the sample to the means (A) for loading a test sample and comparing degrees of color development on a plurality of the detecting element (E) having different detection sensitivities.

Prediction of ovulation day has an important meaning for a couple who wish for a child. Such a prediction is effected by detecting a peak of blood or urine luteinizing hormone (LH) concentration (LH surge) during a menstrual cycle. Since the LH surge is a phenomenon which can be detected only on a specific day during a menstrual cycle, it is necessary to check it periodically. Also, since ovulation occurs 18 to 24 hours after the LH surge, it is necessary to specify accurate date of the LH surge quickly.

However, basal LH level in the follicular or luteal phase and concentration of LH at its peak stage also differ individually. That is, the LH concentration in urines fluctuates depending on each individual or test sample within the range of 50 IU/l or below in the follicular or luteal phase and from 100 to 400 IU/l at the time of the LH surge.

In general, simple semi-quantitative assay of LH is carried out by a hemagglutination test or an EIA technique. In the case of the hemagglutination test, however, it is necessary to perform serial dilution of each test sample for semi-quantitative assay, thus requiring measurement of all of the diluted samples. In the case of the EIA, it cannot be carried out quickly or easily because, in this assay system, semi-quantitative assay is attained by firstly performing reaction of a test sample together with a plurality of LH standard solutions having different concentrations and then comparing the resulting color densities.

Also, a gold colloid agglutination method is known as a simple qualitative LH assay system. However, when the amount of LH is measured by the gold colloid agglutination method with a detection sensitivity of 100 IU/l, samples collected in the previous and following days of the LH peak are judged positive in some cases when the LH peak concentration is high, thus causing difficulty in specifying the LH surge. In addition, since results of this assay is judged by visual observation of changes in color from red of the gold colloid to grey by agglutination, judgement of the results becomes vague when concentration of the LH peak is low, thus causing difficulty in specifying the LH surge.

When a urine LH level is measured using a semi-quantitative assay device of the present invention having three detecting elements (E) with detection sensitivities of 50, 100 and 200 IU/l, the LH concentration in a sample can be divided correctly into measuring ranges of <50 IU/l, 50 to 100 IU/l, 100 to 200 IU/l and >200 IU/l. As the results, the LH surge can be specified correctly taking individual basal LH level into consideration, by simply adding a test sample to the sample-loading means (A) and observing the detecting element (E).

The assay device of the present invention is also useful for the detection of HB virus infection which requires quick diagnosis. In order to grasp conditions of activated HB virus correctly, it is necessary to identify and determine HBs antigens quickly by a simple assay process. In addition, periodical measurement of HBs antigens in and after HB virus infection is effective for the judgement of the proliferation of HB virus, transitional onset of chronic hepatitis and therapeutic effects.

In addition to virus antigens, measurement of anti-HBs antibody titers is also useful for the judgement of morbid states. Periodical quantitative measurement of anti-HBs antibody titers will render possible judgement of the state of the titers, that is, whether they are originated from past virus infection or they are presently increasing titers due to recent virus infection or they are presently decreasing titers due to recovery from the recent infection.

The specific binding assay device of the present invention renders possible quick and simple monitoring of periodical changes in the amount of substances to be assayed. In addition, prior art assay processes are possessed of a possible danger of causing secondary infection when the aforementioned measurement of HB virus and the like is carried out, because these processes require handling of serum samples collected from virus-infected patients. Such a risk can be avoided by the use of the assay device of the present invention, because measurement can be effected by simply adding a test sample to the sample-loading means (A).

Thus, some examples of the application of the assay device of the present invention have been described in the foregoing. The inventive assay device can also be applied effectively to the measurement of other measuring items in which quick and simple quantitative or semi-quantitative assay is required for correct judgement of the assay results.

The specific binding assay device of the third aspect of the present invention also includes the following variations.

As has been described in the foregoing, the assay device shown in Fig. 15 has a set of three units each unit consisting of the means (B) for locating specific binding substances and the means (C) for locating a detecting element. By changing types of specific binding substances and the like to be included in each of these units, a specific binding assay device can be produced by which a plurality of substances to be assayed contained in a single test sample can be measured simultaneously. Similar type of variations may also be obtained from the devices shown in Figs. 13 (I) and (II) and Figs. 14 (I), (II) and (III).

For example, a variation device for use in the simultaneous measurement of a plurality of substances to be assayed in a single test sample may be derived from the assay device shown in Fig. 15, in which a first unit consisting of the means (B-1) for locating specific binding substances and the means (C-1) for locating a detecting element detects CEA, a second unit consisting of the means (B-2) and (C-2) detects AFP and a third unit consisting of the means (B-3) and (C-3) detects hCG.

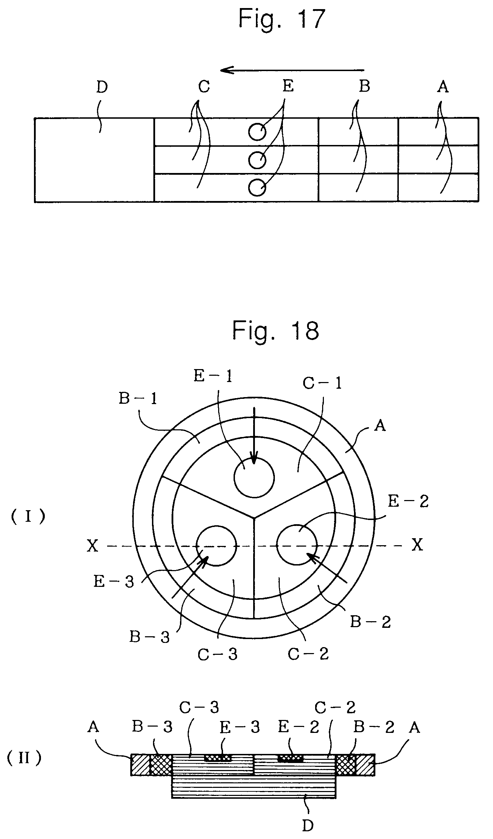

Also included in the third aspect of the present invention is a specific binding assay device which can measure a plurality of test samples simultaneously. As shown in Fig. 17, such a device has a plurality of independent units, each unit having at least the means (A) for loading a test sample, the means (B) for locating specific binding substances and the means (C) for locating a detecting element arranged in continuous manner. Such a type of device is useful especially when simultaneous assay of a number of samples is required, for example, in the case of mass examination. This device may also be made into variation types, for example, in which the means (B) for locating specific binding substances and the means (C) for locating a detecting element occupy the same area.

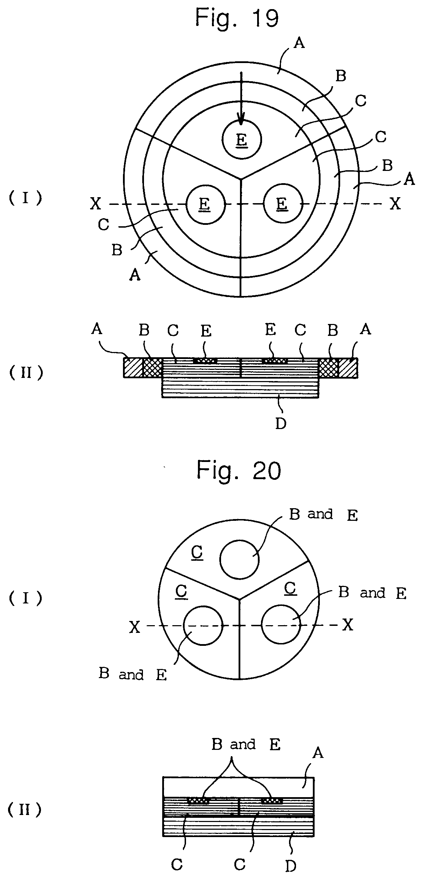

Devices shown in Figs. 18 and 19 are examples of other variations of the assay device of the third aspect of the present invention in which the means (C) for locating a detecting element and the means (D) for absorbing liquid materials have a vertical relationship. In each of these figures, (I) shows a plan view of the device and (II) shows a sectional view at the x-x line.

Each of the aforementioned devices of the third aspect of the present invention can be used suitably for the practice of the assay process of the first or second aspect of the present invention and, in addition to the advantages described in the foregoing, is characterized in that a specific binding assay can be effected only by a step for loading a test sample and a step for judging the results. In addition, results of the assay can be judged correctly by any person by visual observation when a dye or the like is used as the labeling substance.

When an additional step such as B/F separation is required, a device in which a liquid material such as a loaded test sample travels in vertical direction may be produced for use in the practice of the first or second aspect of the present invention. Fig. 20 shows an example of such a device which is a variation of the aforementioned device for use in the measurement of a plurality of substances to be assayed in a single test sample, in which the means (B) for locating specific binding substances and the means (C) for locating a detecting element occupy the same area. In Fig. 20, (I) shows a plan view of the device and (II) shows a sectional view at the x-x line of (I).

EXAMPLES

The following examples are provided to further illustrate the present invention. It is to be understood, however, that the examples are for purpose of illustration only and are not intended as a definition of the limits of the invention.

(Example 1) Measurement of urine hCG (part 1)

(1) Production of assay device

An assay device shown in Fig. 15 (I) was produced in the following manner.
(a) Preparation of dye-labeled anti-hCG$\beta$ antibody
A 0.5 ml portion of a phosphate-buffered saline (PBS, 0.067 M, pH 6.4) containing 100 $\mu$g of mouse monoclonal anti-hCG$\beta$ antibody (Mochida Pharmaceutical Co., Ltd.) was mixed with 0.5 ml of a suspension of a disperse dye (Foron Brilliant Blue, available from SANDOZ K.K.) in water (absorbance at 650 nm was about 200), and the mixture was incubated overnight at 4°C with shaking. The thus incubated mixture was diluted with 4.5 ml of PBS (pH 6.4) and centrifuged at 15,000 rpm for 10 minutes. The resulting precipitate was resuspended in 5.5 ml of PBS (pH 6.4) and the suspension was centrifuged at 15,000 rpm for 10 minutes. Thereafter, the thus obtained precipitate was suspended in 5.5 ml of a stock solution [0.5% bovine serum albumin (BSA), 4% saccharose and 0.1% sodium azide dissolved in PBS (pH 6.4)] to obtain the dye-labeled anti-hCG$\beta$ antibody of interest which was stored at 4°C until its use.
(b) Preparation of means (A) for loading a test sample
A cellulose filter paper for chromatography use (Advantech Toyo, No. 526, 0.7 mm in thickness) was cut using a cutter into a piece having a length (direction of liquid flow which is parallel to the orientation of cellulose fibers) of 10 mm and a width (vertical direction of the liquid flow) of 17 mm. Each of the thus prepared filter paper pieces was impregnated with 100 $\mu$l of PBS (pH 6.4) solution containing 0.3% BSA and 0.1% Tween 20, followed by drying at 45°C.
(c) Preparation of means (B) for locating specific binding substances
The dye-labeled mouse monoclonal anti-hCG$\beta$ antibody prepared in the above step (a) was diluted by a factor of 8 with a buffer A (10% normal rabbit serum, 4% saccharose and 0.5% BSA dissolved in 0.067 M PBS, pH 6.4). Separately from this, mouse monoclonal anti-hCG$\beta$ antibody (not labeled) was dissolved in the buffer A to a final concentration of 0, 2 or 8 $\mu$g/ml. Each of the unlabeled antibody solutions thus prepared was mixed with the diluted sample of the labeled antibody with a mixing ratio of 1:1 (v:v) to obtain three solutions. Thereafter, a polyester non-woven fabric (10 x 5 mm, 0.5 mm in thickness) was impregnated with 25 $\mu$l of each of the three solutions, followed by drying at 45°C.
In this manner, three pieces of polyester non-woven fabric to be used as the means (B-1), (B-2) and (B-3) for locating specific binding substances were obtained, each of which containing the above two types of anti-hCG$\beta$ antibodies in such a mixing ratio that the detection sensitivity of the detecting element (E-1), (E-2) or (E-3), which will be described later, is adjusted to 10 hCG IU/l, 40 hCG IU/l or 160 hCG IU/l.
(d) Preparation of film containing detecting element (E) (means (C) for locating a detecting element)
A 1 $\mu$l portion of a rabbit polyclonal anti-hCG antibody solution (500 $\mu$g/ml, containing 1,000 $\mu$g/ml of BSA) was spotted on the central point of a nitrocellulose film (Advantech Toyo; pore size, 5 $\mu$m; 25 x 5 mm; thickness, 0.16 mm). After drying at room temperature, the resulting film was subjected to blocking by soaking it in PBS (pH 6.4) containing 0.5% BSA and 0.1% Tween 20, followed by drying on a filter paper. Two additional films were prepared in the same manner.
(e) Assembly of assay device
The means (A), (B) and (C) prepared above and a liquid-absorbing means (D) were assembled into an assay device in the following manner to reproduce the cross-sectional view shown in Fig. 11 (II). The means (C-1), (C-2) and (C-3) for locating a detecting element were fixed in parallel at 1 mm intervals on an adhesive sheet to be used as a supporting material (F). One cut edge of each of the means (B-1), (B-2) and (B-3) for locating specific binding substances was connected with one cut edge of each of the means (C-1), (C-2) and (C-3) in such a manner that the connected portion had an overlapping area of 1.5 mm. Next, a cut edge of a cellulose filter paper (Advantech Toyo; No. 585; 10 x 17 mm; 0.8 mm in thickness) to be used as a means (D) for absorbing liquid materials was connected with the other cut edge of each of the means (C-1), (C-2) and (C-3) in such a manner that the connected portion had an overlapping area of 1.5 mm. Thereafter, a cut edge of the means (A) for loading a test sample was contacted with the other cut edge of each of the means (B-1), (B-2) and (B-3).

After arranging these means in this way, they were covered with a water repellent paper and pressed lightly with a roller to ensure their fixation to the supporting material (F). In this manner, the assay device of Fig. 15 (I) having three detecting elements (E-1, E-2 and E-3) was assembled. A total of 6 devices were produced for future use.

In this instance, detection sensitivity and signal intensity (color density) of the assay device are controlled by the amount of the unlabeled mouse monoclonal anti-hCG$\beta$ antibody.

(2) Measurement

(a) Test sample
Urine samples containing 0, 10, 40, 100, 200 and 500 IU/l of hCG were used as test samples.
(b) Measuring techniques
A 180 $\mu$l portion of each of the above urine samples was added to the means (A) for loading a test sample of each of the 6 assay devices produced in (1).

The hCG in the thus loaded urine sample found its way into the means (B) for locating specific binding substances where the hCG was linked to the dye-labeled or unlabeled mouse monoclonal anti-hCG$\beta$ antibody and then into the means (C) for locating a detecting element where the antibody-linked hCG was further linked to the rabbit polyclonal anti-hCG antibody which has been immobilized on the detecting element (E).

Thereafter, a signal (color) originated from the dye-labeled mouse monoclonal anti-hCG$\beta$ antibody was measured at the detecting element (E).

In this instance, excess amounts of the urine sample, the dye-labeled mouse monoclonal anti-hCG$\beta$ antibody and the unlabeled mouse monoclonal anti-hCG$\beta$ antibody were absorbed and maintained in the means (D) for absorbing liquid materials.
(c) Judgement of the results
Results of the color development at the detecting elements (E-1, E-2 and E-3) of the assay device after loading of each urine sample are shown in Table 2 and Fig. 15.

In Table 2, "+" and "++" indicate the presence of colored spot ("++" means more intensive color density than "+"), and "-" indicates the absence of colored spot. In Fig. 15, (I) is an example of the assay device to which a urine sample containing 0 IU/l of hCG is added, (II) is an example of the assay device to which a urine sample containing 10 IU/l of hCG is added, (III) is an example of the assay device to which a urine sample containing 40 or 100 IU/l of hCG is added, (IV) is an example of the assay device to which a urine sample containing 200 IU/l of hCG is added and (V) is an example of the assay device to which a urine sample containing 500 IU/l of hCG is added.

Table 2

| Results of urine hCG measurement (part 1) | | | | | | |
|---|---|---|---|---|---|---|
| | Amount of hCG in urine sample (IU/l) | | | | | |
| | 0 | 10 | 40 | 100 | 200 | 500 |
| Detecting elements | | | | | | |
| E-1 | - | + | + + | + + | + + | + + |
| E-2 | - | - | + | + | + + | + + |
| E-3 | - | - | - | - | + | + + |
| Relation to Fig.15 | (I) | (II) | (III) | (III) | (IV) | (V) |

(Example 2) Measurement of urine hCG (part 2)

(1) Production of assay device

An assay device shown in Fig. 15 (I) was produced in the following manner.

(a) Preparation of dye-labeled anti-mouse IgG antibody

Dye-labeled anti-mouse IgG antibody was prepared by repeating the step (a) of Example 1 (1) except that 0.5 ml of PBS containing 200 μg/ml of a rabbit anti-mouse IgG antibody (available from Dako Patts A/S) was used instead of the mouse monoclonal anti-hCGβ antibody. The thus prepared antibody sample was stored at 4°C until its use.

(b) Preparation of means (A) for loading a test sample

The means (A) was prepared in the same manner as described in Example 1 (1) (b).

(c) Preparation of means (B) for locating specific binding substances

The dye-labeled anti-mouse IgG antibody prepared in the above step (a) was diluted by a factor of 8 with the aforementioned buffer A. Separately from this, mouse monoclonal anti-hCGβ antibody (not labeled) was dissolved in the buffer A to a final concentration of 0.2, 2.0 or 8.0 μg/ml. Each of the anti-hCGβ antibody solutions thus prepared was mixed with the diluted sample of the labeled anti-mouse IgG antibody with a mixing ratio of 1:1 (v:v) to obtain three solutions. Thereafter, a polyester non-woven fabric (10 x 5 mm, 0.5 mm in thickness) was impregnated with 25 μl of each of the three solutions, followed by drying at 45°C.

In this manner, three pieces of polyester non-woven fabric to be used as the means (B-1), (B-2) and (B-3) for locating specific binding substances were obtained, each of which containing the dye-labeled anti-mouse IgG antibody and the mouse monoclonal anti-hCGβ antibody in such a mixing ratio that the detection sensitivity of the detecting element (E-1), (E-2) or (E-3), which will be described later, is adjusted to 10 hCG IU/l, 40 hCG IU/l or 160 hCG IU/l.

(d) Preparation of film containing detecting element (E) (means (C) for locating a detecting element)

The means (C) was prepared in the same manner as described in Example 1 (1) (d).

(e) Assembly of assay device

The step of Example 1 (1) (e) was repeated.

In this instance, detection sensitivity of the assay device is controlled by the amount of the mouse monoclonal anti-hCGβ antibody.

(2) Measurement

(a) Test sample

Urine samples containing 0, 10, 40, 100, 200 and 500 IU/l of hCG were used as test samples.

(b) Measuring techniques

A 180 μl portion of each of the above urine samples was added to the means (A) for loading a test sample of each of the 6 assay devices produced in (1).

The hCG in the thus loaded urine sample found its way into the means (B) for locating specific binding substances where the hCG was linked, or not, to the dye-labeled anti-mouse IgG antibody via the mouse monoclonal anti-hCGβ antibody and then into the means (C) for locating a detecting element where the antibody-linked hCG or free hCG was further linked to the rabbit polyclonal anti-hCG antibody which has been immobilized on the detecting element (E).

Thereafter, a signal (color) originated from the dye-labeled anti-mouse IgG antibody was measured at the detecting element (E).

In this instance, excess amounts of the urine sample, the dye-labeled anti-mouse IgG antibody and the mouse monoclonal anti-hCGβ antibody were absorbed and maintained in the means (D) for absorbing liquid materials.

(c) Judgement of the results

Results of the color development at the detecting elements (E-1, E-2 and E-3) of the assay device after loading of each urine sample are shown in Table 3 and Fig. 15.

In Table 3, "+" and "++" indicate the presence of colored spot ("++" means more intensive color density than "+"), and "-" indicates the absence of colored spot. In Fig. 15, (I) is an example of the assay device to which a urine sample containing 0 IU/l of hCG is added, (II) is an example of the assay device to which a urine sample containing 10 IU/l of hCG is added, (III) is an example of the assay device to which a urine sample containing 40 or 100 IU/l of hCG is added, (IV) is an example of the assay device to which a urine sample containing 200 IU/l of hCG is added and (V) is an example of the assay device to which a urine sample containing 500 IU/l of hCG is added.

EP 0 516 095 A2

Table 3

| Results of urine hCG measurement (part 2) | | | | | | |
|---|---|---|---|---|---|---|
| | Amount of hCG in urine sample (IU/l) | | | | | |
| | 0 | 10 | 40 | 100 | 200 | 500 |
| Detecting elements | | | | | | |
| E-1 | - | + | + + | + + | + + | + + |
| E-2 | - | - | + | + | + + | + + |
| E-3 | - | - | - | - | + | + + |
| Relation to Fig.15 | (I) | (II) | (III) | (III) | (IV) | (V) |

(Example 3) Measurement of serum AFP

(1) Production of assay device

An assay device shown in Fig. 15 (I) was produced in the following manner.

(a) Preparation of mouse monoclonal anti-human AFP antibody No. 1 labeled with a dye

Dye-labeled mouse monoclonal anti-human AFP antibody No. 1 was prepared in the same manner as described in Example 1 (1) (a), except that 0.5 ml of PBS containing 100 $\mu$g of mouse monoclonal anti-human AFP antibody No. 1 (Mochida Pharmaceutical Co., Ltd.) and 0.5 ml of a disperse dye (Kayaron Fast Rubin, available from Nippon Kayaku Co., Ltd.). were used. The thus prepared antibody sample was stored at 4°C until its use.

(b) Preparation of means (A) for loading a test sample

A piece of glass fiber filter paper (Advantech Toyo, GC-50, 10 x 15 mm, 0.2 mm in thickness) was impregnated with 60 $\mu$l of PBS solution (pH 6.4) containing 0.3% BSA and 0.1% Tween 20, followed by drying at 45°C.

(c) Preparation of means (B) for locating specific binding substances

The dye-labeled mouse monoclonal anti-human AFP antibody No. 1 prepared in the above step (a) was diluted by a factor of 8 with the aforementioned buffer A. Separately from this, mouse monoclonal anti-AFP antibody No. 2 (Mochida Pharmaceutical Co., Ltd.) was dissolved in the buffer A to a final concentration of 0, 4 or 6 $\mu$g/ml. Each of the antibody No, 2 solutions thus prepared was mixed with the diluted sample of the labeled antibody No. 1 with a mixing ratio of 1:1 (v:v) to obtain three solutions. Thereafter, a polyester non-woven fabric (10 x 5 mm, 0.5 mm in thickness) was impregnated with 25 $\mu$l of each of the three solutions, followed by drying at 45°C.

In this manner, three pieces of polyester non-woven fabric to be used as the means (B-1), (B-2) and (B-3) for locating specific binding substances were obtained, each of which containing the above two types of anti-AFP antibodies in such a mixing ratio that the detection sensitivity of the detecting element (E-1), (E-2) or (E-3), which will be described later, is adjusted to 5 ng AFP/ml, 100 ng AFP/ml or 500 ng AFP/ml.

(d) Preparation of film containing detecting element (E) (means (C) far locating detecting elements)

A nitrocellulose film (Advantech Toyo; pore size, 5 $\mu$m; 25 x 17 mm; thickness, 0.16 mm) was divided into three independent (not communicable) portions by scratching two lines at 5 mm intervals on the surface of the film using an edge of a plastic plate having a thickness of 1.0 mm.

Next, a 1 $\mu$l portion of a 500 ng/ml solution of the mouse monoclonal anti-AFP antibody No. 2 was spotted on the central point of each of the three divided portions to be used as detecting elements (E-1), (E-2) and (E-3). After drying, the resulting film was soaked in PBS solution (pH 6.4) containing 0.5% BSA and 0.1% Tween 20 and then dried again. In this way, a film having three detecting elements (E-1, E-2 and E-3) was obtained as the means (C) for locating detecting elements).

(e) Assembly of assay device

The means (A), (B) and (C) prepared above and a liquid-absorbing means (D) were assembled into an assay device in the following manner to reproduce the cross-sectional view shown in Fig. 11 (II). The means (C) for locating detecting elements were fixed on a pressure sensitive adhesive sheet to be used as a supporting material (F). One cut edge of each of the means (B-1), (B-2) and (B-3) for locating specific binding substances was connected with one cut edge of the means (C) in such a manner that

28

the connected portion had an overlapping area of 1.5 mm. Next, a cut edge of a cellulose filter paper (Advantech Toyo; No. 585; 10 x 17 mm; 0.8 mm in thickness) to be used as a means (D) for absorbing liquid materials was connected with the other cut edge of the means (C) in such a manner that the connected portion had an overlapping area of 1.5 mm. Thereafter, a cut edge of the means (A) for loading a test sample was contacted with the other cut edge of each of the means (B-1), (B-2) and (B-3).

After arranging these means in this way, they were covered with a water repellent paper and pressed lightly with a roller to ensure their fixation to the supporting material (F). In this manner, the assay device of Fig. 15 (I) having three detecting elements (E-1, E-2 and E-3) was assembled. A total of 5 devices were produced for future use.

In this instance, detection sensitivity of the assay device is controlled by the amount of the mouse monoclonal anti-AFP antibody No. 2 (not immobilized).

(2) Measurement

(a) Test sample

Serum samples containing 0, 5, 100, 500 and 1000 ng/ml of AFP were used as test samples.

(b) Measuring techniques

A 180 $\mu$l portion of each of the above serum samples was added to the means (A) for loading a test sample of each of the 5 assay devices produced in (1).

The AFP in the thus loaded serum sample found its way into the means (B) for locating specific binding substances where the AFP was linked to the dye-labeled mouse monoclonal anti-human AFP antibody No. 1 and/or the unlabeled mouse monoclonal anti-human AFP antibody No. 2 and then into the means (C) for locating detecting elements where AFP molecules which were not linked to the mouse monoclonal anti-human AFP antibody No. 2 in the means (B) were linked to the mouse monoclonal anti-human AFP antibody No. 2 which has been immobilized on the detecting element (E).

Thereafter, a signal (color) originated from the dye-labeled mouse monoclonal anti-human AFP antibody No. 1 was measured at the detecting element (E).

In this instance, excess amounts of the serum sample and AFP-linked or AFP-free portions of the dye-labeled mouse monoclonal anti-human AFP antibody No. 1 and the unlabeled mouse monoclonal anti-human AFP antibody No. 2 were absorbed and maintained in the means (D) for absorbing liquid materials.

(c) Judgement of the results

Results of the color development at the detecting elements (E-1, E-2 and E-3) of the assay device after loading of each serum sample are shown in Table 4 and Fig. 15.

In Table 4, "+" and "++" indicate the presence of colored spot ("++" means more intensive color density than "+"), and "-" indicates the absence of colored spot. In Fig. 15, (I) is an example of the assay device to which a serum sample containing 0 ng/ml of AFP is added, (II) is an example of the assay device to which a serum sample containing 5 ng/ml of AFP is added, (III) is an example of the assay device to which a serum sample containing 100 ng/ml of AFP is added, (IV) is an example of the assay device to which a serum sample containing 500 ng/ml of AFP is added and (V) is an example of the assay device to which a serum sample containing 1000 ng/ml of AFP is added.

Table 4

| Results of serum AFP measurement | | | | | |
|---|---|---|---|---|---|
| | Amount of AFP in serum sample (ng/ml) | | | | |
| | 0 | 5 | 100 | 500 | 1000 |
| Detecting elements | | | | | |
| E-1 | - | + | + + | + + | + + |
| E-2 | - | - | + | + + | + + |
| E-3 | - | - | - | + | + + |
| Relation to Fig.15 | (I) | (II) | (III) | (IV) | (V) |

(Example 4) Measurement of urine estrogen (part 1)

(1) Production of assay device

An assay device shown in Fig. 15 (I) was produced in the following manner. In this instance, each of the means (B) for locating specific binding substances and the means (C) for locating detecting element has two identical units.

(a) Preparation of 17-carboxymethylestrone-BSA

17-Carboxymethylestrone was conjugated with BSA (molar ratio of 17-carboxymethylestrone to BSA, 11:1) in accordance with the mixed anhydride method (Erlanger, B.F. *et al., Method in Immunochemistry*, ed. by William, C.A., Vol.1, pp.141-151, Academic Press New York, 1968), and the resulting 17-carboxymethylestrone-BSA conjugate was purified using Sephadex G-25.

(b) Preparation of 17-carboxymethylestrone-BSA labeled with gold colloid

A 0.2 mg portion of the 17-carboxymethylestrone-BSA prepared in the above step (a) was added to 20 ml of a colloidal gold solution (gold colloid particle size, 10 nm; available from Biocell Research Laboratory) which has been adjusted to pH 7.4 with 0.2 M potassium carbonate solution. After 10 minutes of stirring at room temperature, 0.5 ml of 0.1% polyethylene glycol (PEG) 6000 was added to the mixture, followed by 10 minutes of stirring at room temperature. The thus treated mixture was subjected to centrifugation at 15,000 rpm for 60 minutes, and the resulting precipitate was suspended in 10 ml of 0.1 M Tris-HCl buffer (pH 7.6) containing 0.3% BSA and 0.25% PEG 6000. After 60 minutes of centrifugation at 15,000 rpm, the resulting precipitate was suspended in 4 ml of a buffer B (0.3% BSA, 0.25% PEG 6000 and 4% saccharide dissolved in 0.1 M Tris-HCl, pH 7.6) to obtain the 17-carboxymethylestrone-BSA labeled with gold colloid which was stored at 4°C until its use.

(c) Preparation of means (A) for loading a test sample

A piece of glass fiber filter paper (Advantech Toyo, GC-50, 10 x 11 mm, 0.2 mm in thickness) was impregnated with 70 $\mu$l of 0.1 M Tris-HCl solution (pH 7.6) containing 0.3% BSA and 0.25% PEG 6000, followed by drying at 45°C.

(d) Preparation of means (B) for locating specific binding substances

The gold colloid-labeled 17-carboxymethylestrone-BSA prepared in the above step (b) was diluted by a factor of 8 with the aforementioned buffer B. Separately from this, mouse monoclonal anti-estriol ($E_3$) antibody (Mochida Pharmaceutical Co., Ltd.) was dissolved in the buffer B to a final concentration of 0 or 16 $\mu$g/ml. Each of the antibody solutions thus prepared was mixed with the diluted sample of the labeled 17-carboxymethylestrone-BSA with a mixing ratio of 1:1 (v:v) to obtain two solutions. Thereafter, two pieces of a glass fiber filter paper (Advantech Toyo, GC-50, 10 x 5 mm, 0.25 mm in thickness) were impregnated with 25 $\mu$l of each of the two solutions respectively, followed by drying at 45°C.

In this manner, two pieces of glass fiber filter paper to be used as the means (B-1) and (B-2) for locating specific binding substances were obtained, each of which containing the gold colloid-labeled 17-carboxymethylestrone-BSA and the mouse monoclonal anti-$E_3$ antibody in such a mixing ratio that the detection sensitivity of the detecting element (E-1) or (E-2), which will be described later, is adjusted to 1 $\mu$g $E_3$/ml or 10 $\mu$g $E_3$/ml.

(e) Preparation of film containing detecting element (E) (means (C) for locating detecting elements)

A 1 $\mu$l portion of a solution containing 200 $\mu$g/ml of mouse monoclonal anti-$E_3$ antibody (Mochida Pharmaceutical Co., Ltd.) and 1,000 $\mu$g/ml of BSA was spotted on the central point of a nitrocellulose film (Advantech Toyo; pore size, 5 $\mu$m; 25 x 5 mm; thickness, 0.16 mm). After drying at room temperature, the resulting film was subjected to blocking by soaking it in 0.1 M Tris-HCl solution (pH 7.6) containing 0.5% BSA and 0.25% PEG 6000, followed by drying. A total of two films were prepared in this manner.

(f) Assembly of assay device

The means (A), (B) and (C) prepared above and a liquid-absorbing means (D) were assembled into an assay device in the following manner to reproduce the cross-sectional view shown in Fig. 11 (II). The means (C-1) and (C-2) for locating detecting elements were fixed in parallel at 1 mm interval on a pressure sensitive adhesive sheet to be used as a supporting material (F). One cut edge of each of the means (B-1) and (B-2) for locating specific binding substances was connected with one cut edge of each of the means (C-1) and (C-2) in such a manner that the connected portion had an overlapping area of 1.5 mm. Next, a cut edge of a cellulose filter paper (Advantech Toyo; No. 585; 10 x 17 mm; 0.8 mm in thickness) to be used as a means (D) for absorbing liquid materials was connected with the other cut edge of each of the means (C-1) and (C-2) in such a manner that the connected portion had an overlapping area of 1.5 mm. Thereafter, a cut edge of the means (A) for loading a test sample was contacted with the other cut edge of each of the means (B-1) and (B-2).

After arranging these means in this way, they were covered with a water repellent paper and pressed lightly with a roller to ensure their fixation to the supporting material (F). In this manner, the assay device of Fig. 15 (I) having two detecting elements (E-1 and E-2) was assembled. A total of 5 devices were produced for future use.

In this instance, detection sensitivity of the assay device is controlled by the amount of the mouse monoclonal anti-$E_3$ antibody (not immobilized).

(2) Measurement

(a) Test sample

Urine samples containing 0, 0.5, 1.0, 5.0 and 10 $\mu$g/ml of $E_3$ were used as test samples.

(b) Measuring techniques

A 120 $\mu$l portion of each of the above urine samples was added to the means (A) for loading a test sample of each of the 5 assay devices produced in (1).

When the urine sample entered the means (B) for locating specific binding substances, the gold colloid-labeled 17-carboxymethylestrone-BSA prepared in the above step (b) and the mouse monoclonal anti-$E_3$ antibody (Mochida Pharmaceutical Co., Ltd.) both included in the means (B) were dissolved in the urine sample. The urine sample containing the thus dissolved substances then entered the means (C) for locating detecting elements where a portion of the gold colloid-labeled 17-carboxymethylestrone-BSA, which did not bind to the mouse monoclonal anti-$E_3$ antibody molecules (not immobilized), and the $E_3$ contained in the urine sample underwent competitive binding to the mouse monoclonal anti-$E_3$ antibody which has been immobilized on the detecting element (E).

Thereafter, a signal (color of gold colloid) originated from the gold colloid-labeled 17-carboxymethylestrone-BSA was measured at the detecting element (E).

In this instance, excess amounts of the urine sample and the gold colloid-labeled 17-carboxymethylestrone-BSA linked or not linked to the mouse monoclonal anti-$E_3$ antibody were absorbed and maintained in the means (D) for absorbing liquid materials.

(c) Judgement of the results

Results of the color development at the detecting elements (E-1 and E-2) of the assay device after loading of each urine sample are shown in Table 5.

In Table 5, "+" indicates the presence of colored spot and "-" indicates the absence of colored spot.

Table 5

| Results of urine estrogen measurement (part 1) | | | | | |
|---|---|---|---|---|---|
| | Amount of $E_3$ in urine sample ($\mu$g/ml) | | | | |
| | 0 | 0.5 | 1.0 | 5.0 | 10 |
| Detecting elements | | | | | |
| E-1 | + | + | - | - | - |
| E-2 | + | + | + | + | - |

(Example 5) Measurement of urine estrogen (part 2)

(1) Production of assay device

An assay device shown in Fig. 15 (I) was produced in the following manner. In this instance, each of the means (B) for locating specific binding substances and the means (C) for locating detecting element has two identical units.

(a) Preparation of mouse monoclonal anti-$E_3$ antibody labeled with gold colloid

Gold colloid-labeled mouse monoclonal anti-$E_3$ antibody was prepared in the same manner as in Example 4 (1) (b) except that mouse monoclonal anti-$E_3$ antibody (Mochida Pharmaceutical Co., Ltd.) was used instead of the 17-carboxymethylestrone-BSA. The thus obtained labeled antibody sample was stored at 4°C until its use.

(b) Preparation of means (A) for loading a test sample

The means (A) for loading a test sample was prepared in the same manner as in Example 4 (1) (c).

(c) Preparation of means (B) for locating specific binding substances

The gold colloid-labeled mouse monoclonal anti-$E_3$ antibody prepared in the above step (a) was diluted by a factor of 8 with the aforementioned buffer B. Separately from this, mouse monoclonal anti-$E_3$ antibody (Mochida Pharmaceutical Co., Ltd.) was dissolved in the buffer B to a final concentration of 0 or 12 $\mu$g/ml. Each of the antibody solutions thus prepared was mixed with the diluted sample of the labeled mouse monoclonal anti-$E_3$ antibody with a mixing ratio of 1:1 (v:v) to obtain two solutions. Thereafter, two pieces of a glass fiber filter paper (Advantech Toyo, GC-50, 10 x 5 mm, 0.25 mm in thickness) were impregnated with 25 $\mu$l of each of the two solutions respectively, followed by drying at 45°C.

In this manner, two pieces of glass fiber filter paper to be used as the means (B-1) and (B-2) for locating specific binding substances were obtained, each of which containing the gold colloid-labeled mouse monoclonal anti-$E_3$ antibody and the unlabeled mouse monoclonal anti-$E_3$ antibody in such a mixing ratio that the detection sensitivity of the detecting element (E-1) or (E-2), which will be described later, is adjusted to 1 $\mu$g $E_3$/ml or 10 $\mu$g $E_3$/ml.

(d) Preparation of film containing detecting element (E) (means (C) for locating detecting elements)

A 1 $\mu$l portion of PBS solution containing 200 $\mu$g/ml of the 17-carboxymethylestrone-BSA was spotted on the central point of a nitrocellulose film (Advantech Toyo; pore size, 5 $\mu$m; 25 x 5 mm; thickness, 0.16 mm). After drying at room temperature, the resulting film was subjected to blocking by soaking it in 0.1 M Tris-HCl solution (pH 7.6) containing 0.5% BSA and 0.25% PEG 6000, followed by drying. A total of two films were prepared in this manner.

(e) Assembly of assay device

By repeating the step of Example 4 (1) (f), an assay device having two detecting elements (E-1 and E-2) was assembled. A total of 5 devices were produced for future use.

In this instance, detection sensitivity of the assay device is controlled by the amount of the unlabeled mouse monoclonal anti-$E_3$ antibody.


(2) Measurement

(a) Test sample

Urine samples containing 0, 0.5, 1.0, 5.0 and 10 $\mu$g/ml of $E_3$ were used as test samples.

(b) Measuring techniques

A 120 $\mu$l portion of each of the above urine samples was added to the means (A) for loading a test sample of each of the 5 assay devices produced in (1).

When the urine sample entered the means (B) for locating specific binding substances, the gold colloid-labeled mouse monoclonal anti-$E_3$ antibody prepared in the above step (a) and the unlabeled mouse monoclonal anti-$E_3$ antibody (Mochida Pharmaceutical Co., Ltd.) both included in the means (B) were dissolved in the urine sample. The urine sample containing the thus dissolved substances then entered the means (C) for locating detecting elements where portions of the gold colloid-labeled mouse monoclonal anti-$E_3$ antibody molecules and unlabeled mouse monoclonal anti-$E_3$ antibody molecules, which did not bind to the $E_3$ contained in the urine sample, underwent competitive binding to the 17-carboxymethylestrone-BSA which has been immobilized on the detecting element (E).

Thereafter, a signal (color of gold colloid) originated from the gold colloid-labeled mouse monoclonal anti-$E_3$ antibody was measured at the detecting element (E).

In this instance, excess amounts of the urine sample and the $E_3$-linked (or not linked) gold colloid-labeled mouse monoclonal anti-$E_3$ antibody and the unlabeled mouse monoclonal anti-$E_3$ antibody were absorbed and maintained in the means (D) for absorbing liquid materials.

(c) Judgement of the results

Results of the color development at the detecting elements (E-1 and E-2) of the assay device after loading of each urine sample are shown in Table 6.

In Table 6, "+" indicates the presence of colored spot and "-" indicates the absence of colored spot.

Table 6

| Results of urine estrogen measurement (part 2) | | | | | |
|---|---|---|---|---|---|
| | Amount of $E_3$ in urine sample ($\mu$g/ml) | | | | |
| | 0 | 0.5 | 1.0 | 5.0 | 10 |
| Detecting elements | | | | | |
| E-1 | + | + | - | - | - |
| E-2 | + | + | + | + | - |

(Example 6) Measurement of serum HBs antibody titers

(1) Production of assay device

An assay device shown in Fig. 15 (I) was produced in the following manner. In this instance, each of the means (B) for locating specific binding substances and the means (C) for locating detecting element has two identical units.

(a) Preparation of HBs antigen labeled with a dye

Dye-labeled HBs antigen was prepared in the same manner as in Example 1 (1) (a), except that 0.5 ml of PBS solution containing 200 $\mu$g/ml of a purified HBs antigen preparation (blood plasma origin, available from Cheil Sugar & Co. Ltd.) was used instead of the mouse monoclonal anti-hCG$\beta$ antibody. The thus obtained labeled antibody sample was stored at 4°C until its use.

(b) Preparation of means (A) for loading a test sample

A piece of glass fiber filter paper (Advantech Toyo, GC-50, 10 x 11 mm, 0.16 mm in thickness) was impregnated with 70 $\mu$l of PBS solution (pH 6.4) containing 0.3% BSA and 0.1% Tween 20, followed by drying at 45°C.

(c) Preparation of means (B) for locating specific binding substances

The dye-labeled HBs antigen prepared in the above step (a) was diluted by a factor of 8 with the aforementioned buffer A. Separately from this, unlabeled HBs antigen (yeast origin recombinant HBs) was dissolved in the buffer A to a final concentration of 0 or 10 $\mu$g/ml. Each of the unlabeled antigen solutions thus prepared was mixed with the diluted sample of the dye-labeled HBs antigen with a mixing ratio of 1:1 (v:v) to obtain two solutions. Thereafter, a piece of a polyester non-woven fabric (10 x 5 mm, 0.5 mm in thickness) was impregnated with 25 $\mu$l of each of the two solutions, followed by drying at 45°C.

In this manner, two pieces of polyester non-woven fabric to be used as the means (B-1) and (B-2) for locating specific binding substances were obtained, each of which containing the labeled and unlabeled HBs antigens in such a mixing ratio that the detection sensitivity of the detecting element (E-1) or (E-2), which will be described later, becomes positive when a test sample has 16 times or more titers measured by passive hemagglutination (PHA) or has 128 times or more titers measured by passive hemagglutination (PHA).

(d) Preparation of film containing detecting element (E) (means (C) for locating detecting elements)

A 1 $\mu$l portion of a PBS solution containing 247 $\mu$g/ml of the HBs antigen (yeast origin recombinant HBs) was spotted on the central point of a nitrocellulose film (Advantech Toyo; pore size, 5 $\mu$m; 10 x 5 mm; thickness, 0.16 mm). After drying, the resulting film was subjected to blocking by soaking it in PBS solution (pH 7.2) containing 0.5% casein and 0.1% Tween 20, and the soaked film was then dried on a filter paper. A total of two films were prepared in this manner.

(e) Assembly of assay device

By repeating the step of Example 4 (1) (f), an assay device having two detecting elements (E-1 and E-2) was assembled. A total of 5 devices were produced for future use.

In this instance, detection sensitivity of the assay device is controlled by the amount of the free (not immobilized) HBs antigen (yeast origin recombinant HBs).

(2) Measurement

(a) Test sample

An HBs-negative serum sample and HBs-positive serum samples 1 (titers by PHA, 16 times), 2 (titers by PHA, 64 times), 3 (titers by PHA, 128 times) and 4 (titers by PHA, 512 times) were used as test samples.

(b) Measuring techniques

A 180 $\mu$l portion of each of the above serum samples was added to the means (A) for loading a test sample of each of the 5 assay devices produced in (1).

When the serum sample entered the means (B) for locating specific binding substances, the anti-HBs antibody contained in the sample bound to the dye-labeled HBs antigen prepared in the above step (a) and/or the unlabeled HBs antigen (yeast origin recombinant HBs). The serum sample containing the thus linked substances then entered the means (C) for locating detecting elements where a portion of the anti-HBs antibody molecules having unreacted HBs antigen-combining sites bound to the HBs antigen (yeast origin recombinant HBs) which has been immobilized on the detecting element (E).

Thereafter, a signal (color) originated from the dye-labeled HBs antigen bound via the anti-HBs antibody in the serum sample to the HBs antigen immobilized on the detecting element (E) was measured.

(c) Judgement of the results

Results of the color development at the detecting elements (E-1 and E-2) of the assay device after loading of each serum sample are shown in Table 7.

In Table 7, "+" and "+ +" indicate the presence of colored spot ("+ +" means more intensive color density than "+") and "-" indicates the absence of colored spot.

Table 7

| Results of serum HBs antibody titer measurement | | | | | |
|---|---|---|---|---|---|
| | Anti-HBs antibody titer in serum sample (times) | | | | |
| | (negative) | 16 | 64 | 128 | 512 |
| Detecting elements | | | | | |
| E-1 | - | + | + + | + + | + + |
| E-2 | - | - | - | + | + + |

(Example 7) Measurement of serum HBs

(1) Production of assay device

An assay device shown in Fig. 15 (I) was produced in the following manner.

(a) Preparation of mouse monoclonal anti-HBs antibody No. 1 labeled with a dye

Dye-labeled mouse monoclonal anti-HBs antibody No. 1 was prepared in the same manner as in Example 1 (1) (a), except that mouse monoclonal anti-HBs antibody No. 1 (Mochida Pharmaceutical Co., Ltd.) was used instead of the mouse monoclonal anti-hCG$\beta$ antibody. The thus obtained labeled antibody sample was stored at 4°C until its use.

(b) Preparation of means (A) for loading a test sample

The means (A) was prepared in the same manner as in Example 1 (1) (b).

(c) Preparation of means (B) for locating specific binding substances

The dye-labeled mouse monoclonal anti-HBs antibody No. 1 prepared in the above step (a) was diluted by a factor of 10 with the aforementioned buffer A. Separately from this, unlabeled mouse monoclonal anti-HBs antibody No. 2 (Mochida Pharmaceutical Co., Ltd.) was dissolved in the buffer A to a final concentration of 0, 2 or 8 $\mu$g/ml. Each of the unlabeled anti-HBs antibody No. 2 solutions thus prepared was mixed with the diluted sample of the dye-labeled anti-HBs antibody No. 1 with a mixing ratio of 1:1 (v:v) to obtain three solutions. Thereafter, a piece of a polyester non-woven fabric (10 x 5 mm, 0.5 mm in thickness) was impregnated with 25 $\mu$l of each of the three solutions, followed by drying at 45°C.

In this manner, three pieces of polyester non-woven fabric to be used as the means (B-1), (B-2) and (B-3) for locating specific binding substances were obtained, each of which containing the dye-labeled mouse monoclonal anti-HBs antibody No. 1 and unlabeled mouse monoclonal anti-HBs antibody No. 2 in such a mixing ratio that the detection sensitivity of the detecting element (E-1), (E-2) and (E-3), which will be described later, respectively become 5 ng HBs/ml, 100 ng HBs/ml and 1000 ng HBs/ml.

(d) Preparation of film containing detecting element (E) (means (C) for locating detecting elements)

The means (C) was prepared in the same manner as in Example 1 (1) (d), except that mouse monoclonal anti-HBs antibody No. 2 (Mochida Pharmaceutical Co., Ltd.) was used instead of the rabbit polyclonal anti-hCG antibody. A total of two films were prepared in this manner.

(e) Assembly of assay device

The step of Example 1 (1) (e) was repeated to assemble an assay device.

In this instance, detection sensitivity of the assay device is controlled by the amount of the free (not immobilized) mouse monoclonal anti-HBs antibody No. 2.

(2) Measurement

(a) Test sample

Five serum samples listed below were collected from a patient infected with HBV and used as test samples.

Sample 1: serum sample collected before HBV infection

Sample 2: serum sample collected 10 weeks after HBV infection

Sample 3: serum sample collected 20 weeks after HBV infection

Sample 4: serum sample collected 30 weeks after HBV infection

Sample 5: serum sample collected 40 weeks after HBV infection

(b) Measuring techniques

A 180 $\mu$l portion of each of the above serum samples was added to the means (A) for loading a test sample of each of the assay devices produced in (1).

When the serum sample entered the means (B) for locating specific binding substances, the HBs antigen contained in the sample bound to the dye-labeled mouse monoclonal anti-HBs antibody No. 1 and/or the unlabeled mouse monoclonal anti-HBs antibody No. 2. The serum sample containing the thus bound substances then entered the means (C) for locating detecting elements where a portion of the HBs antigen molecules which did not bind to the mouse monoclonal anti-HBs antibody No. 2 in the means (B) was linked to the mouse monoclonal anti-HBs antibody No. 2 that has been immobilized on the detecting element (E).

Thereafter, a signal (color) originated from the dye-labeled mouse monoclonal anti-HBs antibody No. 1 was measured at the detecting element (E).

(c) Judgement of the results

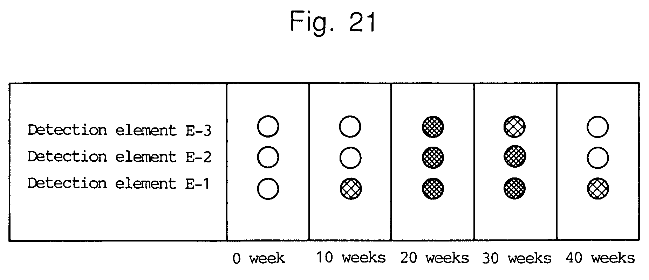

Results of the color development at the detecting elements (E-1, E-2 and E-3) of the assay device after loading of each serum sample are shown in Fig. 21.

(Example 8) Comparison of the inventive assay process with prior art assay processes in terms of urine LH measurement

Amounts of LH in urine samples collected from a healthy woman during a menstrual cycle were measured using the assay process of the present invention (semi-quantitative), and the results were compared with other results of the measurement of the same urine samples using three prior art processes (gold colloid agglutination test for qualitative assay, hemagglutination test for semi-quantitative assay and radioimmunoassay for quantitative assay).

(1) Production of assay device

An assay device shown in Fig. 15 (I) was produced in the following manner.

(a) Preparation of mouse monoclonal anti-LH$\alpha$ antibody labeled with a dye

Dye-labeled mouse monoclonal anti-LH$\alpha$ antibody was prepared in the same manner as in Example 1 (1) (a), except that mouse monoclonal anti-LH$\alpha$ antibody (Mochida Pharmaceutical Co., Ltd.) was used instead of the mouse monoclonal anti-hCG$\beta$ antibody. The thus obtained labeled antibody sample was stored at 4°C until its use.

(b) Preparation of means (A) for loading a test sample

The means (A) was prepared in the same manner as in Example 1 (1) (b).

(c) Preparation of means (B) for locating specific binding substances

The dye-labeled mouse monoclonal anti-LH$\alpha$ antibody prepared in the above step (a) was diluted by a factor of 10 with the aforementioned buffer A. Separately from this, unlabeled mouse monoclonal anti-LH$\beta$ antibody (Mochida Pharmaceutical Co., Ltd.) was dissolved in the buffer A to a final concentration of 0.1, 1.2 or 6 $\mu$g/ml. Each of the unlabeled anti-LH$\beta$ antibody solutions thus prepared was mixed with the diluted sample of the dye-labeled anti-LH$\alpha$ antibody with a mixing ratio of 1:1 (v:v) to obtain three solutions. Thereafter, a piece of a polyester non-woven fabric (10 x 5 mm, 0.5 mm in thickness) was impregnated with 25 $\mu$l of each of the three solutions, followed by drying at 45°C.

In this manner, three pieces of polyester non-woven fabric to be used as the means (B-1), (B-2) and (B-3) for locating specific binding substances were obtained, each of which containing the dye-labeled mouse monoclonal anti-LH$\alpha$ antibody and the unlabeled mouse monoclonal anti-LH$\beta$ antibody in such a mixing ratio that the detection sensitivity of the detecting element (E-1), (E-2) and (E-3), which will be described later, respectively become 50 IU of LH/l, 100 IU of LH/l and 200 IU of LH/l.

(d) Preparation of film containing detecting element (E) (means (C) for locating detecting elements)

The means (C) was prepared in the same manner as in Example 1 (1) (d), except that mouse monoclonal anti-LH$\beta$ antibody (Mochida Pharmaceutical Co., Ltd.) was used instead of the rabbit polyclonal anti-hCG antibody. A total of two films were prepared in this manner.

(e) Assembly of assay device

The step of Example 1 (1) (e) was repeated to assemble an assay device.

In this instance, detection sensitivity of the assay device is controlled by the amount of the free (not immobilized) mouse monoclonal anti-LH$\beta$ antibody.

(2) Measurement

(a) Inventive process

A 180 $\mu$l portion of each of the aforementioned urine samples was added to the means (A) for loading a test sample of the assay device produced in (1), and color densities at the detecting elements (E-1, E-2 and E-3) were observed after 10 minutes of the sample-loading to perform semi-quantitative assay.

(b) Prior art processes

Gold colloid agglutination test was carried out by dissolving a freeze-dried preparation of anti-LH antibody immobilized gold colloid in an appropriate buffer, adding an appropriate amount of each urine sample to the resulting solution and then judging results of the reaction after 30 minutes of the sample-loading by visual observation of the color of gold colloid. In this instance, a sample was judged positive (+) when changes in the gold colloid solution from red to gray was visually detected.

Hemagglutination test was carried out using an assay kit (HI-GONAVIS available from Mochida Pharmaceutical Co., Ltd.). That is, each urine sample was diluted with a dilution solution attached to the assay kit by a factor of 1 (no dilution), 4 or 8 and then subjected to the agglutination assay. A sample was judged positive (+) when aggregation was observed.

Radioimmunoassay was carried out using an assay kit (SPAC-S LH kit, available from DAIICHI RADIOISOTOPE LABS., Ltd.) in accordance with the manual of the kit.

(3) Comparison of the assay results

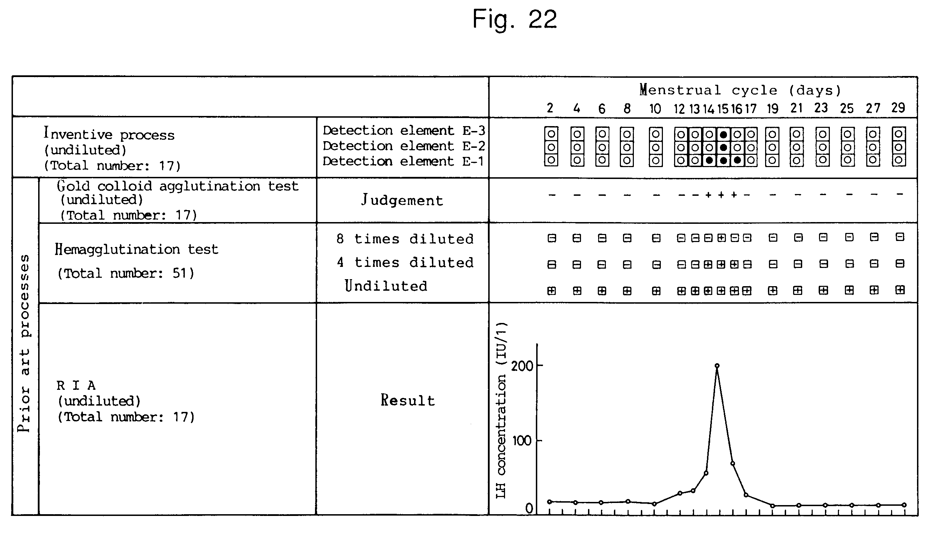

The results are summarized in Fig. 22.

As shown in the figure, results obtained by the four assay processes coincided well with one another. On the basis of these results, it can be said that the assay process of the present invention which is effected by the use of the inventive assay device can be used efficiently for accurate semi-quantitative assay, in spite of the simple handling with only one sample required for one test.

(Example 9) Comparison of the inventive assay process with prior art assay processes in terms of urine hCG measurement

The assay process of the present invention (semi-quantitative) was compared with two prior art processes (semi-quantitative EIA and quantitative EIA) with regard to the measurement of urine hCG.

(1) Measurement by the inventive process

Urine samples containing 0 to 320 IU/l of hCG were assayed in accordance with the process of Example 1.

36

(2) Measurement by prior art processes

(a) Semi-quantitative assay

Each urine sample containing a certain amount of hCG was diluted with a hCG-free dilution solution (pooled urine from a plurality of male persons) by a factor of 2 or 4, and the original urine sample and the diluted samples were checked for their hCG contents using an EIA kit for qualitative hCG assay (hCG TestPack available from Abbott Laboratories; detection sensitivity, 25 IU/l) in accordance with the manual of the assay kit. A sample was judged positive ( + ) when it developed color.

(b) Quantitative assay

An EIA kit for hCG assay use (Gonachrome, available from Mochida Pharmaceutical Co., Ltd.) was used. In this kit, anti-hCG antibody is immobilized in a sealed glass test tube in which a freeze-dried preparation of peroxidase-labeled anti-hCG antibody is also included. The reaction starts when an hCG-containing sample is transferred into the glass test tube and the contents are mixed. After completion of the reaction and subsequent washing, a solution consisting of hydrogen peroxide as a substrate and 3,3',5,5'-tetramethylbenzidine as a color-producing reagent is added to the tube, and absorbance of the developed hCG-corresponding color in the substrate/color-producing reagent mixture solution is measured quantitatively at 650 nm.

(3) Comparison of the assay results

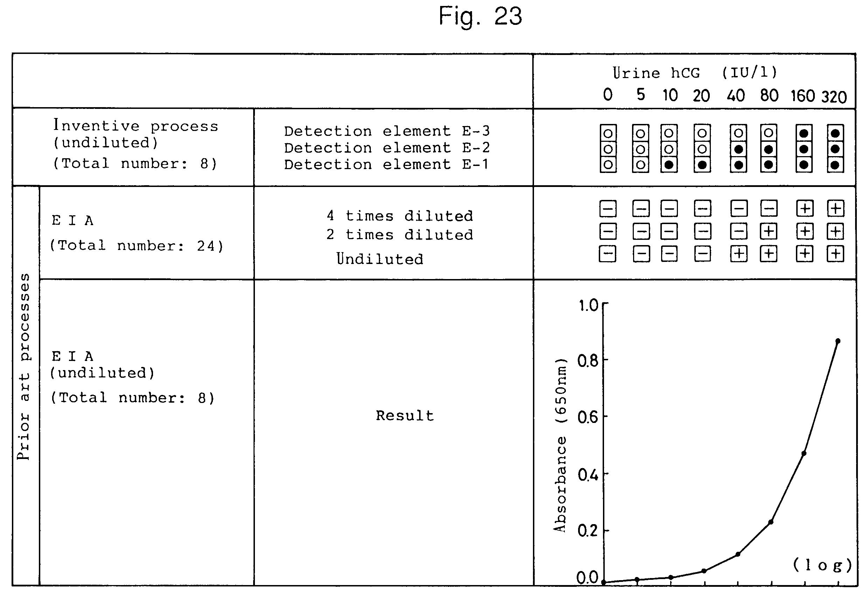

The results are summarized in Fig. 23.

As shown in the figure, results obtained by the three assay processes coincided well with one another. On the basis of these results, it can be said that the assay process of the present invention which is effected by the use of the inventive assay device can be used efficiently for accurate semi-quantitative assay, in spite of the simple handling with only one sample required for one test.

Thus, it is apparent that there has been provided, in accordance with the present invention, an assay process which is especially effective for semi-quantitative assay at a high sensitivity, by which assay results similar to the case of the use of a diluted test sample can be obtained through only one step of loading a sample as it is, and in which any desired detection sensitivity can be selected at will without causing decrease (or increase) in the maximum intensity of signals. It is apparent also that there has been provided an assay device useful for the practice of the assay process.

According to the present invention, accurate measurement can be made easily by any person who has no special knowledge about medical techniques.

Also, according to the present invention, possible danger of causing infection to a technician due to pollution with test samples can be reduced greatly, because a preliminary step for sample dilution is not necessary and a loaded sample does not leak from the assay device.

Moreover, according to the present invention, semi-quantitative assay can be effected more precisely than the case of prior art processes, because detection sensitivity and signal intensity of the inventive assay device can be set to various levels easily.

In addition, according to the present invention, a plurality of test items or of test samples can be measured simultaneously and easily.

Although some preferred embodiments have been described, many modifications and variations may be made thereto in the light of the above teachings. It is therefore to be understood that within the scope of the appended claims, the invention may be practiced otherwise than as specifically described.

## Claims

1. A process for specific binding assay in which a substance in a test sample to be assayed is detected qualitatively or quantitatively making use of specific binding reaction, which comprises including a specified substance in an assay system and, by the effect of the presence of said specified substance, decreasing amounts of a labeled material to be measured as an index of the amount of said substance to be assayed.

2. The process for specific binding assay according to claim 1 wherein said specified substance is a specific binding substance which has a specific affinity for said substance to be assayed.

3. The process for specific binding assay according to claim 1 wherein said specified substance is an analogue of said substance to be assayed.

4. The process for specific binding assay according to any one of the claims 1 to 3 wherein said labeled material is formed through labeling of said specific binding substance which has a specific affinity for said substance to be assayed.

5. The process for specific binding assay according to claim 1 or 2 wherein said labeled material is formed through labeling of a substance which is capable of binding itself to said specific binding substance having a specific affinity for said substance to be assayed without interfering binding reaction of said substance to be assayed with said specific binding substance.

6. The process for specific binding assay according to claim 4 wherein said process comprises the steps of:
   allowing a substance (a) in a test sample to be assayed to undergo binding reaction simultaneously or successively with a specific binding substance (b) having a specific affinity for said substance (a) to be assayed and with a labeled specific binding substance (e) which is a compound resulting from binding of a labeling substance (d) to a specific binding substance (c) that does not interfere with said specific binding substance (b) at the time of binding reaction with said substance (a) to be assayed;
   allowing a binding substance (f), which is a compound resulting from the above binding reaction of said substance (a) to be assayed with said labeled specific binding substance (e), to undergo binding reaction with said specific binding substance (b) in an immobilized form or with an immobilized specific binding substance (g) that does not interfere with said labeled specific binding substance (e) but interferes with said specific binding substance (b) at the time of binding reaction with said substance (a) to be assayed, thereby immobilizing said binding substance (f); and
   reading off signals originated from the labeling substance (d) in the immobilized binding substance (f).

7. The process for specific binding assay according to claim 4 wherein said process comprises the steps of:
   allowing a substance (h) in a test sample to be assayed having a plurality of sites related to one specific binding reaction to undergo binding reaction simultaneously or successively with a specific binding substance (i) having a specific affinity for the site related to one specific binding reaction of said substance (h) to be assayed, with a labeled specific binding substance (k) which is a compound resulting from binding of the labeling substance (d) to a specific binding substance (j) having a specific affinity for the site related to one specific binding reaction and with an immobilized specific binding substance (l) having a specific affinity for the site related to one specific binding reaction, thereby immobilizing at least a portion of said substance (h) to be assayed; and
   reading off signals originated from the labeling substance (d) in said labeled specific binding substance (k) bound to the immobilized substance (h) to be assayed.

8. The process for specific binding assay according to claim 4 wherein said process comprises the steps of:
   allowing the substance (a) in a test sample to be assayed to undergo binding reaction with a labeled specific binding substance (n) which is a compound resulting from binding of the labeling substance (d) to a specific binding substance (m) having a specific affinity for said substance (a) to be assayed;
   allowing an immobilized specific binding substance (o), which has a specific affinity for said substance (a) to be assayed and is not interfered with the binding reaction of said substance (a) to be assayed with said labeled specific binding substance (n), to undergo binding reaction with a binding substance (p) which is a compound resulting from the binding of said substance (a) to be assayed to said labeled specific binding substance (n), with an analogue (q) of the substance to be assayed that does not bind to said labeled specific binding substance (n) but binds to said specific binding substance (o) and, if exist, with unbound substance (a) to be assayed, thereby immobilizing said binding substance (p) and said analogue (q); and
   reading off signals originated from the labeling substance (d) in the immobilized binding substance (p).

9. The process for specific binding assay according to claim 4 wherein said process comprises the steps of:
   allowing a labeled specific binding substance (s), which is a compound resulting from binding of

38

EP 0 516 095 A2

the labeling substance (d) to a specific binding substance (r) having a specific affinity for the substance (a) to be assayed, to undergo binding reaction simultaneously or successively with the substance (a) in a test sample to be assayed and with an analogue (t) of the substance to be assayed that interferes binding reaction of said labeled specific binding substance (s) with said substance (a) to be assayed;

allowing a binding substance (u) which is a compound resulting from the above binding reaction of said substance (a) to be assayed with said labeled specific binding substance (s) and, if exist, unbound substance (a) to undergo binding reaction with an immobilized specific binding substance (v) that does not interfere with said labeled specific binding substance (s) and does not bind to said analogue (t) of the substance to be assayed at the time of binding reaction with said substance (a) to be assayed, thereby immobilizing said binding substance (u); and

reading off signals originated from the labeling substance (d) in the immobilized binding substance (u).

10. The process for specific binding assay according to claim 4 wherein said process comprises the steps of:

allowing the substance (a) in a test sample to be assayed to undergo binding reaction simultaneously or successively with a specific binding substance (w) having a specific affinity for said substance (a) to be assayed and with a labeled specific binding substance (y) which is a compound resulting from binding of the labeling substance (d) to a specific binding substance (x) that interferes with said specific binding substance (w) at the time of binding reaction with said substance (a) to be assayed;

allowing a binding substance (z) which is a compound resulting from the above binding reaction of said substance (a) to be assayed with said specific binding substance (w) and a binding substance ($\alpha$) which is a compound resulting from the above binding reaction of said substance (a) to be assayed with said labeled specific binding substance (y) to undergo binding reaction with an immobilized specific binding substance ($\beta$) that does not interfere with said specific binding substance (w) or said labeled specific binding substance (y) at the time of binding reaction with said substance (a) to be assayed, thereby immobilizing said binding substances (z) and ($\alpha$); and

reading off signals originated from the labeling substance (d) in the immobilized binding substance ($\alpha$).

11. The process for specific binding assay according to claim 5 wherein said process comprises the steps of:

allowing a specific binding substance ($\gamma$) having a specific affinity for the substance (a) to be assayed to undergo binding reaction simultaneously or successively with the substance (a) in a test sample to be assayed and with a labeled specific binding substance ($\epsilon$) which is a compound resulting from binding reaction of the labeling substance (d) with a specific binding substance ($\delta$) that has a specific affinity for said specific binding substance ($\gamma$) and does not interfer with said substance (a) to be assayed at the time of binding reaction with said specific binding substance ($\gamma$);

allowing a binding substance ($\zeta$) which is a combined material resulting from the above binding reaction of said substance (a) to be assayed with said specific binding substance ($\gamma$) and said labeled specific binding substance ($\epsilon$) and a binding substance ($\eta$) which is a combined material resulting from the above binding reaction of said substance (a) to be assayed with said specific binding substance ($\gamma$) to undergo binding reaction with an immobilized specific binding substance ($\theta$) that has a specific affinity for said substance (a) to be assayed and is not interfered with the binding reaction of said substance (a) to be assayed with said specific binding substance ($\gamma$), thereby immobilizing said binding substances ($\zeta$) and ($\eta$); and

reading off signals originated from the labeling substance (d) in the immobilized binding substance ($\zeta$).

12. A process for specific binding assay in which a substance in a test sample to be assayed is detected qualitatively or quantitatively making use of specific binding reaction, which comprises including a specified substance in an assay system and, by the effect of the presence of said specified substance, increasing amounts of a labeled material to be measured as an index of the amount of said substance to be assayed.

13. The process for specific binding assay according to claim 12 wherein said specified substance is a specific binding substance which has a specific affinity for said substance to be assayed.

39

**14.** The process for specific binding assay according to claim 12 or 13 wherein said labeled material is formed through labeling of said specific binding substance which has a specific affinity for said substance to be assayed.

**15.** The process for specific binding assay according to claim 12 or 13 wherein said labeled material is formed through labeling of a substance which is capable of binding itself to said specific binding substance having a specific affinity for said substance to be assayed without interfering binding reaction of said substance to be assayed with said specific binding substance.

**16.** The process for specific binding assay according to claim 12 or 13 wherein said labeled material is formed through labeling of said substance to be assayed or an analogue thereof.

**17.** The process for specific binding assay according to claim 14 wherein said process comprises the steps of:

allowing a specific binding substance ($x$) having specific affinities for the substance (a) to be assayed and an analogue ( $\iota$) thereof and a labeled specific binding substance ($\mu$) which is a compound resulting from binding of the labeling substance (d) to a specific binding substance ($\lambda$) that interferes with said specific binding substance ($x$) at the time of the binding reaction with said substance (a) to be assayed and said analogue ( $\iota$) to undergo binding reaction simultaneously or successively with the substance (a) in a test sample to be assayed and with immobilized form of the substance (a) to be assayed or of the analogue ( $\iota$), thereby immobilizing at least a portion of said labeled specific binding substance ($\mu$); and

reading off signals originated from the labeling substance (d) in the immobilized labeled specific binding substance ($\mu$).

**18.** The process for specific binding assay according to claim 15 wherein said process comprises the steps of:

allowing a specific binding substance ($\zeta$) having specific affinities for the substance (a) to be assayed and an analogue ($\nu$) thereof to undergo binding reaction simultaneously or successively with the substance (a) in a test sample to be assayed, with immobilized form of the substance (a) to be assayed or of the analogue ($\nu$) and with a labeled specific binding substance ($\rho$) which is a compound resulting from binding of the labeling substance (d) to a specific binding substance ($\pi$) that has a specific affinity for said specific binding substance ($\zeta$) and does not interfere with the substance (a) to be assayed and the analogue ($\nu$) at the time of the binding reaction with the specific binding substance ($\zeta$), thereby immobilizing at least a portion of said labeled specific binding substance ($\rho$) via the specific binding substance ($\zeta$); and

reading off signals originated from the labeling substance (d) in the immobilized labeled specific binding substance ($\rho$).

**19.** The process for specific binding assay according to claim 16 wherein said process comprises the steps of:

allowing the substance (a) in a test sample to be assayed and a labeled substance ($\sigma$) to be assayed which is a compound resulting from binding of the labeling substance (d) to the substance (a) to be assayed or a labeled analogue ($\Phi$) of the substance to be assayed, which is a compound resulting from binding of the labeling substance (d) to an analogue ($\tau$) of the substance (a) to be assayed, to undergo binding reaction simultaneously or successively with a specific binding substance ($\phi$) having specific affinities for the substance (a) to be assayed and the labeled substance ($\sigma$) to be assayed or the labeled analogue ($\Phi$) and with immobilized form of the specific binding substance ($\phi$) or with an immobilized specific binding substance ($\omega$) that interferes with the binding reaction of the specific binding substance ($\phi$) with the substance (a) to be assayed and the labeled substance ($\sigma$) to be assayed or the labeled analogue ($\Phi$) of the substance to be assayed, thereby immobilizing at least a portion of the labeled substance ($\sigma$) to be assayed or the labeled analogue ($\Phi$) of the substance to be assayed; and

reading off signals originated from the labeling substance (d) in the immobilized labeled substance ($\sigma$) to be assayed or the labeled analogue ($\Phi$) of the substance to be assayed.

**20.** A specific binding assay device of a chromatography type useful for effecting the specific binding assay process of claim 6, which comprises a means (A) for loading a test sample, a means (B) for

40

locating specific binding substances, a means (C) for locating a detecting element and a means (D) for absorbing liquid materials in that order, wherein said means (B) contains the specific binding substance (b) and the labeled specific binding substance (e) of claim 6 and said means (C) contains a detecting element (E) in which said specific binding substance (b) or the specific binding substance (g) of claim 6 is immobilized.

21. The specific binding assay device according to claim 20 wherein said means (B) for locating specific binding substances and said means (C) for locating a detecting element are made into one section.

22. The specific binding assay device according to claim 20 or 21 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance (b) and said labeled specific binding substance (e) located in said means (B) and said specific binding substance (b) or said specific binding substance (g) located in said detecting element (E) is contained in each of said units in different amounts.

23. The specific binding assay device according to claim 22 wherein said device has a unit which does not contain said specific binding substance (b) of said means (B).

24. A specific binding assay device of a chromatography type useful for effecting the specific binding assay process of claim 7, which comprises a means (A) for loading a test sample, a means (B) for locating specific binding substances, a means (C) for locating a detecting element and a means (D) for absorbing liquid materials in that order, wherein said means (B) contains the specific binding substance (i) and the labeled specific binding substance (k) of claim 7 and said means (C) contains a detecting element (E) in which the specific binding substance (l) of claim 7 is immobilized.

25. The specific binding assay device according to claim 24 wherein said specific binding substance (i) and said labeled specific binding substance (k) are located in an optional order along the flow direction of a test sample in said means (B).

26. The specific binding assay device according to claim 24 or 25 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance (i) and said labeled specific binding substance (k) located in said means (B) and said specific binding substance (l) located in said detecting element (E) is contained in each of said units in different amounts.

27. The specific binding assay device according to claim 26 wherein said device has a unit which does not contain said specific binding substance (i) of said means (B).

28. A specific binding assay device of a chromatography type useful for effecting the specific binding assay process of claim 8, which comprises a means (A) for loading a test sample, a means (B) for locating specific binding substances, a means (C) for locating a detecting element and a means (D) for absorbing liquid materials in that order, wherein said means (B) contains the labeled specific binding substance (n) and the analogue (q) of the substance to be assayed of claim 8 and said means (C) contains a detecting element (E) in which the specific binding substance (o) of claim 8 is immobilized.

29. The specific binding assay device according to claim 28 wherein said labeled specific binding substance (n) and said analogue (q) of the substance to be assayed are located in an optional order along the flow direction of a test sample in said means (B).

30. The specific binding assay device according to claim 28 or 29 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said labeled specific binding substance (n) and said analogue (q) of the substance to be assayed located in said means (B) and said specific binding substance (o) located in said detecting element (E) is contained in each of said units in different amounts.

31. The specific binding assay device according to claim 30 wherein said device has a unit which does not contain said analogue (q) in said means (B).

32. A specific binding assay device of a chromatography type useful for effecting the specific binding assay process of claim 9, which comprises a means (A) for loading a test sample, a means (B) for locating specific binding substances, a means (C) for locating a detecting element and a means (D) for absorbing liquid materials in that order, wherein said means (B) contains the labeled specific binding substance (s) and the analogue (t) of the substance to be assayed of claim 9 and said means (C) contains a detecting element (E) in which the specific binding substance (v) of claim 9 is immobilized.

33. The specific binding assay device according to claim 32 wherein said labeled specific binding substance (s) and said analogue (t) of the substance to be assayed are located in an optional order along the flow direction of a test sample in said means (B).

34. The specific binding assay device according to claim 32 or 33 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said labeled specific binding substance (s) and said analogue (t) of the substance to be assayed located in said means (B) and said specific binding substance (v) located in said detecting element (E) is contained in each of said units in different amounts.

35. The specific binding assay device according to claim 34 wherein said device has a unit which does not contain said analogue (t) of the substance to be assayed of said means (B).

36. A specific binding assay device of a chromatography type useful for effecting the specific binding assay process of claim 10, which comprises a means (A) for loading a test sample, a means (B) for locating specific binding substances, a means (C) for locating a detecting element and a means (D) for absorbing liquid materials in that order, wherein said means (B) contains the specific binding substance (w) and the labeled specific binding substance (y) of claim 10 and said means (C) contains a detecting element (E) in which the specific binding substance ($\beta$) of claim 10 is immobilized.

37. The specific binding assay device according to claim 36 wherein said specific binding substance (w) and said labeled specific binding substance (y) are located in an optional order along the flow direction of a test sample in said means (B).

38. The specific binding assay device according to claim 36 or 37 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance (w) and said labeled specific binding substance (y) located in said means (B) and said specific binding substance ($\beta$) located in said detecting element (E) is contained in each of said units in different amounts.

39. The specific binding assay device according to claim 38 wherein said device has a unit which does not contain said specific binding substance (w).

40. A specific binding assay device of a chromatography type useful for effecting the specific binding assay process of claim 11, which comprises a means (A) for loading a test sample, a means (B) for locating specific binding substances, a means (C) for locating a detecting element and a means (D) for absorbing liquid materials in that order, wherein said means (B) contains the specific binding substance ($\gamma$) and the labeled specific binding substance ($\epsilon$) of claim 11 and said means (C) contains a detecting element (E) in which the specific binding substance ($\theta$) of claim 11 is immobilized.

41. The specific binding assay device according to claim 40 wherein said specific binding substance ($\gamma$) and said labeled specific binding substance ($\epsilon$) are located in an optional order along the flow direction of a test sample in said means (B).

42. The specific binding assay device according to claim 40 or 41 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance ($\gamma$) and said labeled specific binding substance ($\epsilon$) located in said means (B) and said specific binding substance ($\theta$) located in said means (C) is contained in each of said units in different amounts.

43. A specific binding assay device of a chromatography type useful for effecting the specific binding assay process of claim 17, which comprises a means (A) for loading a test sample, a means (B) for locating specific binding substances, a means (C) for locating a detecting element and a means (D) for absorbing liquid materials in that order, wherein said means (B) contains the specific binding substance ($x$) and the labeled specific binding substance ($\mu$) of claim 17 and said means (C) contains a detecting element (E) in which the substance (a) to be assayed or the analogue ($\tau$) of the substance to be assayed of claim 17 is immobilized.

44. The specific binding assay device according to claim 43 wherein said specific binding substance ($x$) and said labeled specific binding substance ($\mu$) are located in an optional order along the flow direction of a test sample in said means (B).

45. The specific binding assay device according to claim 43 or 44 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance ($x$) and said labeled specific binding substance ($\mu$) located in said means (B) and said substance (a) to be assayed or said analogue ($\tau$) located in said detecting element (E) is contained in each of said units in different amounts.

46. The specific binding assay device according to claim 45 wherein said device has a unit which does not contain said specific binding substance ($x$) of said means (B).

47. A specific binding assay device of a chromatography type useful for effecting the specific binding assay process of claim 18, which comprises a means (A) for loading a test sample, a means (B) for locating specific binding substances, a means (C) for locating a detecting element and a means (D) for absorbing liquid materials in that order, wherein said means (B) contains the specific binding substance ($\varsigma$) and the labeled specific binding substance ($\rho$) of claim 18 and said means (C) contains a detecting element (E) in which the substance (a) to be assayed or the analogue ($\nu$) of claim 18 is immobilized.

48. The specific binding assay device according to claim 47 wherein said specific binding substance ($\varsigma$) and said labeled specific binding substance ($\rho$) are located in an optional order along the flow direction of a test sample in said means (B).

49. The specific binding assay device according to claim 47 or 48 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance ($\varsigma$) and said labeled specific binding substance ($\rho$) located in said means (B)

EP 0 516 095 A2

and said substance (a) to be assayed or said analogue ($\nu$) located in said detecting element (E) is contained in each of said units in different amounts.

50. A specific binding assay device of a chromatography type useful for effecting the specific binding assay process of claim 19, which comprises a means (A) for loading a test sample, a means (B) for locating specific binding substances, a means (C) for locating a detecting element and a means (D) for absorbing liquid materials in that order, wherein said means (B) contains the specific binding substance ($\phi$) and the labeled substance ($\sigma$) or the labeled analogue ($\Phi$) of the substance to be assayed of claim 19 and said means (C) contains immobilized form of said specific binding substance ($\phi$) or of the specific binding substance ($\omega$) of claim 19.

51. The specific binding assay device according to claim 50 wherein said specific binding substance ($\phi$) and said labeled substance ($\sigma$) or said labeled analogue ($\Phi$) are located in an optional order along the flow direction of a test sample in said means (B).

52. The specific binding assay device according to claim 50 or 51 capable of effecting semi-quantitative assay of a substance in a test sample, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where at least one of said specific binding substance ($\phi$) and said labeled substance ($\sigma$) or said labeled analogue ($\Phi$) located in said means (B) and said specific binding substance ($\phi$) or said specific binding substance ($\omega$) located in said detecting element (E) is contained in each of said units in different amounts.

53. The specific binding assay device according to claim 52 wherein said device has a unit which does not contain said specific binding substance ($\phi$) of said means (B).

54. The specific binding assay device according to any one of the claims 20, 21, 24, 28, 32, 36, 40, 43, 47 and 50 wherein said means (A) for loading a test sample and said means (B) for locating specific binding substances are made into one section.

55. The specific binding assay device according to any one of the claims 22, 23, 26, 27, 30, 31, 34, 35, 38, 39, 42, 45, 46, 49, 52 and 53 wherein amounts of said substances are changed by turns.

56. The specific binding assay device according to any one of the claims 20, 21, 24, 25, 28, 29, 32, 33, 36, 37, 40, 41, 43, 44, 47, 48, 50 and 51 which is capable of measuring a plurality of test samples at the same time, wherein said device has a plurality of independent units, each unit having at least a means (A) for loading a test sample, a means (B) for locating specific binding substances and a means (C) for locating a detecting element arranged in continuous manner.

57. The specific binding assay device according to claim 56 wherein said means (A) for loading a test sample and said means (B) for locating specific binding substances are made into one section.

58. The specific binding assay device according to any one of the claims 20, 21, 24, 25, 28, 29, 32, 33, 36, 37, 40, 41, 43, 44, 47, 48, 50 and 51 which is capable of measuring a plurality of substances in one test sample at the same time, wherein said means (A) for loading a test sample exists as a common section, said means (B) for locating specific binding substances and said means (C) for locating a detecting element exist as a plurality of independent units and said means (D) for absorbing liquid materials exists as a common section or is included in said units, where the substance included in each unit is used for the measurement of different substance to be assayed.

44

# Fig. 1

$$\left(\begin{array}{c}\text{Absorption} \\ \text{means (D)}\end{array}\right) \left(\begin{array}{c}\text{Means for locating} \\ \text{detection element} \\ \text{(C)} \\ \text{Detection element} \\ \text{(E)}\end{array}\right) \left(\begin{array}{c}\text{Means for locating} \\ \text{specific binding} \\ \text{substances (B)}\end{array}\right) \left(\begin{array}{c}\text{Means for} \\ \text{loading test} \\ \text{samples (A)}\end{array}\right)$$

① 

b or g

② 

b or g

③ 

b or g

# Fig. 2

$\left(\begin{array}{l}\text{Absorption} \\ \text{means (D)}\end{array}\right)$ $\left(\begin{array}{l}\text{Means for locating} \\ \text{detection element} \\ \text{(C)} \\ \text{Detection element} \\ \text{(E)}\end{array}\right)$ $\left(\begin{array}{l}\text{Means for locating} \\ \text{specific binding} \\ \text{substances (B)}\end{array}\right)$ $\left(\begin{array}{l}\text{Means for} \\ \text{loading test} \\ \text{samples (A)}\end{array}\right)$

## Fig. 3

# Fig. 4

# Fig. 5

EP 0 516 095 A2

# Fig. 6

50

# Fig. 7

$$\left(\begin{array}{c}\text{Absorption} \\ \text{means (D)}\end{array}\right) \left(\begin{array}{c}\text{Means for locating} \\ \text{detection element} \\ \text{(C)} \\ \text{Detection element} \\ \text{(E)}\end{array}\right) \left(\begin{array}{c}\text{Means for locating} \\ \text{specific binding} \\ \text{substances (B)}\end{array}\right) \left(\begin{array}{c}\text{Means for} \\ \text{loading test} \\ \text{samples (A)}\end{array}\right)$$

# Fig. 8

$$\left(\begin{array}{c}\text{Absorption}\\ \text{means (D)}\end{array}\right) \left(\begin{array}{c}\text{Means for locating}\\ \text{detection element}\\ \text{(C)}\\ \text{Detection element}\\ \text{(E)}\end{array}\right) \left(\begin{array}{c}\text{Means for locating}\\ \text{specific binding}\\ \text{substances (B)}\end{array}\right) \left(\begin{array}{c}\text{Means for}\\ \text{loading test}\\ \text{samples (A)}\end{array}\right)$$

① a or ν

② a or ν

③ a or ν

# Fig. 9

## Fig. 10

( I )

Signal strength

Amount (or concentration) of substances to be assayed

( II )

Signal strength

Amount (or concentration) of substances to be assayed

( III )

Signal strength

Amount (or concentration) of substances to be assayed

( IV )

Signal strength

Amount (or concentration) of substances to be assayed

# Fig. 11

# Fig. 12

# Fig. 13

# Fig. 14

(I)

b、e ( and g)

D      B and C     A

(II)

b、e ( and g)

D      B and C     A

(III)

b or g   e (b)   b (e)

C and D   E     B    A

# Fig. 15

# Fig. 16

## Fig. 17

## Fig. 18

( I )

( II )

# Fig. 19

( I )

( II )

# Fig. 20

( I )

( II )

## Fig. 21

| | 0 week | 10 weeks | 20 weeks | 30 weeks | 40 weeks |
|---|---|---|---|---|---|
| Detection element E-3 | ○ | ○ | ⬤ | ⊗ | ○ |
| Detection element E-2 | ○ | ○ | ⬤ | ⬤ | ○ |
| Detection element E-1 | ○ | ⊗ | ⬤ | ⬤ | ⊗ |

EP 0 516 095 A2

# Fig. 22

EP 0 516 095 A2

|  | | Menstrual cycle (days) |
| --- | --- | --- |
|  | | 2  4  6  8  10  12 13 14 15 16 17  19  21  23  25  27  29 |
| Inventive process (undiluted) (Total number: 17) | Detection element E-3 Detection element E-2 Detection element E-1 |  |
| Gold colloid agglutination test (undiluted) (Total number: 17) | Judgement | −  −  −  −  −  − − + + + −  −  −  −  −  −  − |
| Hemagglutination test (Total number: 51) | 8 times diluted |  |
|  | 4 times diluted |  |
|  | Undiluted |  |
| R I A (undiluted) (Total number: 17) | Result |  |

Prior art processes

LH concentration (IU/1)

# Fig. 23

| | | | Urine hCG (IU/l) | | | | | | | |
|---|---|---|---|---|---|---|---|---|---|---|
| | | | 0 | 5 | 10 | 20 | 40 | 80 | 160 | 320 |
| Inventive process (undiluted) (Total number: 8) | Detection element E-3 Detection element E-2 Detection element E-1 | | | | | | | | | |
| Prior art processes | E I A (Total number: 24) | 4 times diluted 2 times diluted Undiluted | | | | | | | | |
| | E I A (undiluted) (Total number: 8) | Result | | | | | | | | |

EP 0 516 095 A2